(19) Europäisches Patentamt
European Patent Office
Office européen des brevets

(11) Publication number: 0 499 337 A1

(12) # EUROPEAN PATENT APPLICATION

(21) Application number: 92200429.6

(51) Int. Cl.[5]: A61K 31/475

(22) Date of filing: 14.02.92

(30) Priority: 14.02.91 EP 91200305

(43) Date of publication of application:
19.08.92 Bulletin 92/34

(84) Designated Contracting States:
AT BE CH DE DK ES FR GB GR IT LI LU MC NL PT SE

(71) Applicant: BROCADES PHARMA B.V.
Elisabethhof 19 P.O. Box 108
NL-2350 AC Leiderdorp(NL)

(72) Inventor: Hegemann, Lutz, c/o Thomas Jefferson University
Medical College, Department of Dermatology
1020 Locust Street Philadelphia PA 19107(US)

(74) Representative: Matulewicz, Emil Rudolf Antonius, Dr. et al
Gist-Brocades NV Patents and Trademarks Department Wateringseweg 1 P.O. Box 1
NL-2600 MA Delft(NL)

(54) **Use of tetra:hydro-thieno-pyrido-indole derivatives for the treatment of skin disorders.**

(57) The use of tetra:hydro-thieno-pyrido-indole derivatives and the pharmaceutically acceptable acid addition salts thereof for preparing a medicament for the treatment of hyperproliferative and/or inflammatory skin disorders is provided.

EP 0 499 337 A1

The present invention relates to preparations containing 7,8,9,10-tetra:hydro-thieno-[3,2-e]-pyrido-[4,3-b]-indole derivatives and pharmaceutically acceptable acid addition salts thereof for the treatment of hyperproliferative and/or inflammatory skin disorders.

BACKGROUND OF THE INVENTION

Skin disorders are frequently occurring afflictions and especially those which are characterized by epidermal hyperproliferation and/or inflammation. Examples thereof include psoriasis, cutaneous tumours primary to the skin, keratosis, ichtyosis, acne, rosacea and dermatitis (all types of eczema). For the treatment of these diseases a large variety of medicines is available. Although many drugs are rather successful in suppressing the symptoms, giving temporary relief of complaints, they cause more or less serious side effects, which may be of systemic or of topical nature and, consequently, restrict the applications of those drugs.

Psoriasis is one of the most common skin disorders, which is reflected by a prevalence rate of about 5% of the population world wide. Clinically, the disease is characterized by hyperkeratosis (skin acanthosis) and epidermal inflammation, which occur in a periodic fashion. For the treatment of psoriasis a variety of drugs, which are administered either topically or systemically or in a combination regimen, is available. These compounds are structurally unrelated and have different mechanisms of action. However, in many cases their healing success is rather disappointing. Furthermore, several drugs evoke severe side effects, especially when applied during long term treatments which are often necessary for the therapy of this chronic disease.

For more than a century the topical application of anthralin is known to be effective in the treatment of psoriasis and, today, the drug is still probably the most widely used. However, it is well documented that anthralin while attenuating lesional inflammation often generates perilesional dermatitis. Furthermore, the drug causes intensive staining of clothing and of the skin and has cosmetic disadvantages [Shroot et al. (1976) in: Psoriasis (Farber et al, ed), Yorke Medical Books, NY, pp. 327-336; McDonald (1983) in: The Chemotherapy of Psoriasis (Baden, ed), Pergamon Press Oxford, pp. 208-210]. Moreover, in numerous studies, anthralin has been proven to be cancerogenic in animal models [Bock and Burns (1963), J. Natl. Canc. Inst. 30: 393-397; Segal et al. (1971), J. Med. Chem. 14: 1152-1154].

Another topically used product is coal tar, which has similar disadvantages as described for anthralin. Also coal tar has been shown to induce skin cancer in animals.

Other drugs used for the treatment of psoriasis include corticosteroids, vitamin D derivatives, retinoids, cyclosporin A and methotrexate. Also photochemotherapy is used.

Corticosteroids, when applied topically over extended periods cause deterioration of the skin [Meyrick Thomas and Black (1985) in: Models in Dermatology (Maibach and Lowe, ed.) Basel, vol 2. pp 30-34].

Vitamin D derivatives can be administered either topically or systemically but have effects on the calcium metabolism resulting in hypercalcemia and decalcification of the bones [de Luca (1976) Am. J. Clin. Nutr., 29: 1258].

Cyclosporin A, retinoids and methotrexate are very potent drugs in the therapy of psoriasis but have severe side effects. Cyclosporin A, initially developed as an immunosuppressive for use in transplantation surgery, is very nephrotoxic, induces hypertension and elevated cholesterol and triglyceride levels [Brit. J. Dermatol. (1990) 122: 365; Fifth International Psoriasis Symposium, July 10-14, 1991, San Fransisco].

Similar side effects were observed with methotrexate, which also is an immunosuppressive and unspecifically inhibits cell proliferation [Roenich and Maibach (1991): Methotrexate, in: Psoriasis, Marcel Dekker New York].

Retinoids have been shown to be teratogenic and, therefore, a possible conception of the patient during therapy must be excluded. Furthermore, the drug is accused of causing hair loss, brittle nails, vision problems and changes of the lipid metabolism [Psoriasis Symposium 1991, as mentioned above]. In order to avoid the severe side effects of the latter drugs, many attempts have been made to develop topical formulations. However, thus far no such formulation showing therapeutic activity has been composed.

At present, severe causes of psoriasis are often treated with systemic photochemotherapy (PUVA, i.e. oral psoralen and UVA light). This therapy bears the risk of causing direct changes in the genomic structure of the patient's cells and is, therefore, potentially mutagenic and cancerogenic.

Since most patients with psoriasis have moderate symptoms the risks and side effects associated with the regimens as described above are not warranted. Therefore, there is a need for an effective and safe form of (local) therapy for the majority of patients afflicted with hyperproliferative and/or inflammatory skin diseases.

In recent years, progress has been made in the field of cellular signal transduction, i.e. the transduction

of extra-cellular information through the membrane, which, subsequently, induces a cellular response. In the regulation of cell proliferation, a crucial role for phosphoinositides is well accepted [Berridge et al. (1985), J. Cell. Sci. Suppl. 3: 187-198]. During signal transduction via the inositide cascade, two second messengers, diacylglycerol and inositol triphosphate, are formed, which, subsequently, activate protein kinase C (PKC). Furthermore, PKC has been identified as the cellular receptor for tumour promoting phorbol esters, such as phorbol-12-myristate-13-acetate (PMA). Therefore, PKC is strongly suggested to play a key role in the regulation of cell proliferation [Nishizuka (1984), Nature 308: 693-698].

As yet, only very limited information about the regulation of keratinocyte proliferation is available. Phorbol esters, when applied to mouse skin in vivo, have been shown to induce epidermal hyperplasia and inflammation [Marks (1983), Carcinogenesis 4: 1465-1470]. In epidermal cell culture, PMA was found to induce cell proliferation and keratinocyte differentiation [Hawley Nelson et al. (1982), Exp. Cell. Res. 137: 155-167]. Furthermore, PKC activity and phorbol ester binding have been detected in human keratinocytes [Snoek et al. (1987), Exp. Cell. Res. 172: 146-157]. Therefore, it seems to be likely that PKC is involved in the regulation of keratinocyte proliferation.

There is also evidence that PKC might be involved in the release of reactive oxygen species from human leucocytes, a key reaction in inflammatory responses [wymann et al. (1987), J. Biol. Chem. 262: 12048-12053]. Since, clinically, psoriasis is characterized by epidermal hyperproliferation and inflammation, PKC is thought to be an important enzyme in the pathophysiology of this disease. PKC is present in human epidermis [Fisher et al. (1987), J. Invest. Dermatol. 89: 484-488] and, indeed, recent studies comparing skin of psoriatic lesions with healthy skin showed differences in activity and distribution of PKC [Horn et al - (1987), J. Invest. Dermatol. 88: 220-222; Inohara et al. (1988), Arch. Dermatol. Res. 280: 454-455].

Gupta et al. [J. Invest. Dermatol. (1988), 91: 486-491] reported that sphingosine inhibited the PMA-induced inflammation, hyperplasia, induction of ornithine decarboxylase and activation of PKC in mouse skin. This is consistent with an earlier report that sphingosine inhibits the generation of reactive oxygen species from human leukocytes in high (cytotoxic) concentration [Wilson et al. (1986), J. Biol. Chem. 261: 12616-12623]. International Patent Application wo 88/01869 has also suggested the use of amphipathic long chain bases, such as sphingosine and sphinganine, for the treatment of inflammation, psoriasis and tumour metastases. However, sphingosine has been shown to inhibit cell differentiation [Merrill et al. (1986), J. Biol. Chem 261: 12610-12615], which would be a major drawback in psoriasis, since in this disease the keratinocytes show increased proliferation and decreased differentiation. Furthermore, sphingosine was found to stimulate fibroblast proliferation [Zhang et al. (1990), J. Biol. Chem. 265: 76-81], reducing its possible therapeutic use in dermatology. Beside other effects on various enzymes, sphingosine is known to inhibit PKC (Hannun et al. (1986), J. Biol. Chem. 261: 12604-12609].

Gupta et al. (see above) suggested the potential of PKC inhibitors in inflammatory diseases, such as psoriasis. However, the investigations with sphingosine clearly demonstrate that this hypothesis cannot lead to the conclusion that all compounds which inhibit PKC might be suitable as drugs for the treatment of psoriasis. Although some of the side effects, such as the stimulation of fibroblast proliferation, have been proven to be mediated by a PKC-independent mechanism, and thus not conflicting the hypothesis of Gupta et al., no PKC inhibitor which might be of use in the treatment of psoriasis has been identified yet.

Another example for this situation is staurosporire, one of the most potent PKC inhibitors [Tamaoki et al. (1986), Biochem. Biophys. Res. Commun. 135: 397-402]. The drug has been described to inhibit inflammatory responses in human neutrophils [Sako et al. (1988), Cancer Res. 48: 4646-4650], but failed to inhibit PMA-induced ornithine decarboxylase activity, a marker enzyme for cell proliferation, in isolated mouse epidermal cells (Kiyoto et al. (1987), Biochem. Biophys. Res. Commun. 148: 740-746]. Moreover, in a study on mouse skin, staurosporine was found to have tumour promoting activity [Yoshizawa et al. (1990), Cancer Res. 50: 4974-4978]. This effect, of course, prevents the clinical use of staurosporine in any indication. The reason for these and many other unwanted effects of staurosporine is thought to be its unselective inhibition of protein kinases by interfering with their ATP binding site, a binding site which is common in many enzymes [Rüegg and Burgess (1989), Trends Pharmacol. Sci. 10: 218-220]. Furthermore, although effectively inhibiting keratinocyte proliferation [Hegemann et al. (1991), Arch. Dermatol. Res. 283: 456-460], the drug causes morphological changes in epidermal cells [Hegemann et al. (1991), J. Invest. Dermatol. 94: 578], a side effect which might correlate to the drug's tumour promoting activity.

Known PKC inhibitors, despite of their effects of keratinocyte proliferation and inflammatory responses in neutrophils, which point towards a usefulness in psoriasis, all seem to have major drawbacks limiting or preventing their clinical use. Interestingly, it has recently been found that the antipsoriatic drug, anthralin, inhibits PKC and might exert its clinical efficacy via this mechanism [Hegemann et al. (1990), Skin Pharmacol. 3: 196]. However, the well documented side effects of anthralin, i.e. the generation of perilesional dermatitis, were also monitored in relevant test systems since the drug induced the release of

the inflammatory mediator, reactive oxygen species, from human neutrophils, when the cells were unstimulated.

7,8,9,10-tetra:hydro-thieno-[3,2-e]-pyrido-[4,3-b]-indole derivatives are the subject of European patents EP-B-0012347 and EP-B-0120439. Initially, these compounds were developed as antidepressant drugs for systemical administration [Glaser and Seidel (1987), Drugs of the Future 12: 562-564]. Later, tiflucarbine and some structural analogues, belonging to the group of tetra:hydro-thieno-pyrido-indole derivatives, were surprisingly found to bind to calmodulin [Schmidt et al. (1990), Eur. J. Pharmacol. 189: 411-418]. Furthermore, tiflucarbine was found to significantly inhibit PKC activity in in-vitro tests [Hegemann et al. (1989), Biol. Chem. Hoppe-Seyler 370: 908].

SUMMARY OF THE INVENTION

The use of 7,8,9,10-tetra:hydro-thieno-[3,2-e]-pyrido-[4,3-b]-indolesof formula I

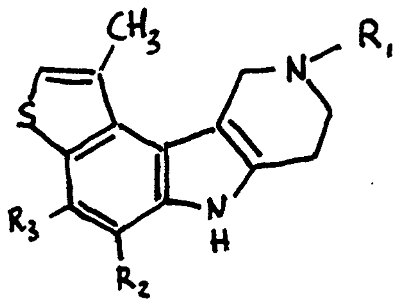

I

(wherein $R_1$ is hydrogen or $C_1$-$C_4$-alkyl;
$R_2$ and $R_3$ may be the same or different, and are selected from hydrogen and halogen) and the pharmaceutically acceptable acid addition salts thereof is provided for preparing a medicament for the treatment of epidermal hyperproliferative and/or inflammatory skin diseases.

Also provided are compositions, which comprise a compound of formula I as mentioned above, for the treatment of hyperproliferative and/or inflammatory skin disorders.

LEGENDS TO THE FIGURES

Figure 1 is a graphical representation of the inhibition of keratinocyte proliferation (HaCaT cells), as measured by the incorporation of [$^3$H]-thymidine and [$^{14}$C]-amino acids into attached cells, by different concentrations of tiflucarbine.

Figure 2 is a graphical representation of the inhibition of keratinocyte proliferation (HaCaT cells), as measured by the total protein content of attached cells, by different concentrations of tiflucarbine.

Figure 3 is a graphical representation of the inhibition of generation of reactive oxygen species (ROS) from human polymorphonuclear leukocytes (PMNL), as measured by lucigenin-amplified chemiluminescence (spontaneous and stimulated after treatment with opsonized zymosan), by different concentrations of tiflucarbine.

Figure 4 is a graphical representation of the inhibition of generation of reactive oxygen species (ROS) from human polymorphonuclear leukocytes (PMNL), as measured by lucigenin-amplified chemiluminescence (spontaneous and stimulated after treatment with the phorbol ester PMA), by different concentrations of tiflucarbine.

Figure 5 is a graphical representation of the effect of different concentrations of tiflucarbine (TFC) and staurosporine on the spontaneous cornified envelope formation by human keratinocytes.

Figure 6 is a graphical representation of the effect of different concentrations of tiflucarbine (TFC) and staurosporine on ionophore-induced cornified envelope formation by human keratinocytes.

Figure 7 is a representation of keratin expression in HaCaT cells, as analyzed by two-dimensional gel electrophoresis for control cells (a), cells grown in the presence of 30 nM staurosporine (c) and 10 $\mu$M tiflucarbine (d).

Figure 8 is a graphical representation of keratin synthesis in HaCaT cells during exposure to staurosporine and tiflucarbine, as determined by density scanning of autoradiographs.

Figure 9 is a representation of the effect of staurosporine (30 nM) on HaCaT cell morphology 42 h (c) and 72 h (d) after plating, compared with control cells 42 h (c) and 72 h (d) after plating.

Figure 10 is a graphical representation of the phosphate incorporation into B-50 and 50 kDa protein, as a function of different concentrations of tiflucarbine.

Figure 11 is a graphical representation of the phosphate incorporation into B-50 and 50 kDa protein, as a function of different concentations of polymyxin B.

Figure 12 is a graphical representation of the effects of several concentrations of tiflucarbine on the release of [$^3$H]-noradreline from rat brain synaptosomes.

Figure 13 is a graphical representation of the effects of several concentrations of cyclosporine A, sphingosine and tiflucarbine on the skin fold thickness after TPA-induced ear edema in mice.

Figure 14 is a graphical representation of the effects of several concentrations of cyclosporine A, sphingosine and tiflucarbine on the skin thickness after TPA-induced hyperproliferation in mouse skin.

Figure 15 is a graphical representation of the effects of several concentrations of tiflucarbine and sphingosine upon systemical administration on ear thickness after TPA-induced ear edema in mice.

## DETAILED DESCRIPTION OF THE INVENTION

It has now been found that tiflucarbine inhibits in a dose-dependent manner the in-vitro proliferation of human keratinocytes, transformed as well as normal (see Examples 1 and 2). Further tiflucarbine does not induce the release of the inflammatory mediator, reactive oxygen species, from human polymorphonuclear leukocytes (PMNL) (see Example 3), this in contrast to anthralin.

The inhibitory effects of tiflucarbine on keratinocyte proliferation and on generation of reactive oxygen species from human polymorphonuclear leukocytes could not have been predicted from:

a. the chemical structure of tiflucarbine;

b. its known therapeutical activity as an anti-depressant;

c. its recently discovered pharmacological activity as a protein kinase C inhibitor and a calmodulin antagonist.

In cytotoxicity tests tiflucarbine has been found to be toxic only in concentrations above 300 $\mu$M (see Example 4). Other PKC inhibitors have been found to be much more toxic in the same tests. A further advantage of tiflucarbine is the rather favourable ratio between the effective and the cytotoxic concentration.

In contrast to other PKC inhibitors, tiflucarbine does not induce morphological changes in transformed keratinocytes, nor does influence keratin expression (see Example 5).

It has further been found that tiflucarbine has no effect on PKC from the central nervous system (see Example 8). The PKC inhibiting activity shown by tiflucarbine is rather specific for PKC, present in the skin.

In animal models tiflucarbine, being administered either topically or systemically, has been found to have a reducing effect on 12-O-tetradecanoylphorbol-13-acetate (TPA) induced ear edema and hyperproliferation in mouse skin (see Examples 8 and 9).

Some of the surprising effects exerted by tiflucarbine, as demonstrated in the examples, have also been found for structurally related compounds.

It has now been found that 7,8,9,10-tetra:hydro-thieno-[3,2-e]-pyrido-[4,3-b]-indoles and their pharmaceutically acceptable acid addition salts can be advantageously used in preparations for the treatment of epidermal hyperproliferative skin disorders, inflammatory skin diseases and diseases of the skin characterized by having an epidermal hyperproliferative and an inflammatory component. These preparations do not cause side-effects as generally observed in common methods for the treatment of the skin disorders as mentioned above.

The 7,8,9,10-tetra:hydro-thieno-[3,2-e]-pyrido-[4,3-b]-indoles and their pharmaceutically acceptable acid addition salts of the present invention are represented by general formula I:

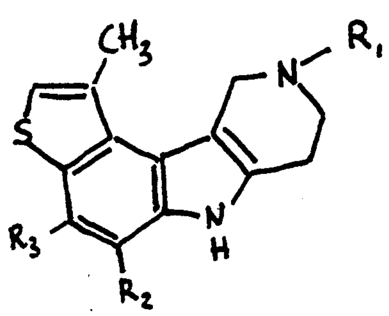

I

wherein $R_1$ is selected from hydrogen or $C_1$-$C_4$-alkyl (straight or branched), methyl or ethyl being the preferred group; and $R_2$ and $R_3$ may be the same or different, and are selected from the group consisting of hydrogen and or a halogen. Preferably the halogen is chosen from fluorine and/or chlorine.

For the preparation of compositions comprising a compound of formula I, a pharmaceutically acceptable acid addition salt thereof, prepared from organic as well as from inorganic acids, can also be used. Examples of said acids include hydrochloric acid, maleic acid, tartaric acid, succinic acid, oxalic acid and preferably lactic acid.

The most preferred compound according to the present invention is tiflucarbine or 1-methyl-9-ethyl-4-fluoro-7,8,9,10-tetra:hydro-thieno-[3,2-e]-pyrido-[4,3-b]-indole lactate.

The dosage-forms, into which the 7,8,9,10-tetra:hydro-thieno-[3,2-e]-pyrido-[4,3-b]-indoles and their pharmaceutically acceptable acid addition salts can be advantageously incorporated for the treatment of the epidermal hyperproliferative and/or inflammatory skin diseases, may be chosen from those as generally known in the pharmaceutical and cosmetic art. The type of dosage-form, that will be chosen, depends of the type of skin disorder and the preferred route of administration. Tablets, capsules and liquid forms can be used for oral administration; solutions, suspensions and emulsions for parenteral administration; and lotions, emulsions (o/w as well as w/o), and ointments for topical application. However, it will be appreciated, that any suitable dosage-form can be used.

For the preparation of the above-mentioned dosage-forms a compound of formula I or a pharmaceutically acceptable salt thereof is mixed with one or more pharmaceutically acceptable excipients or with a suitable vehicle, such as an emulsion or a liquid.

Topical preparations, comprising the 7,8,9,10-tetra:hydro-thieno-[3,2-e]-pyrido-[4,3-b]-indoles and their pharmaceutically acceptable acid addition salts, are preferred for the treatment of the hyperproliferative and/or inflammatory skin disorders. The precise type of skin disease, that has to be treated, will be an important factor in choosing an appropriate vehicle and the components therein. Chronic, dry skin diseases will require the use of a fatty o/w cream, e.g. as described in EP-B-0069423, an ointment or a w/o emulsion as the base into which the active ingredient can be advantageously incorporated. For the treatment of acute, weeping skin disorders, thin emulsions, liniments and aqueous suspensions are preferred bases for incorporation of the active ingredient.

It will be appreciated that the amount of 7,8,9,10-tetra:-hydro-thieno-[3,2-e]-pyrido-[4,3-b]-indoles and their pharmaceutically acceptable acid addition salts that has to be incorporated into the compositions for the effective treatment of the above-mentioned skin disorders will vary according to the route of administration and to the kind and severity of the dermatological disorder, that has to be treated. Generally concentrations of 7,8,9,10-tetra:hydro-thieno-[3,2-e]-pyrido-[4,3-b]-indoles and their pharmaceutically acceptable acid addition salts can range from 0.001 to 20 wt%, the percentages being calculated from the amount of active ingredient, as the base, and from the total weight of the composition. A preferred range, however, is 0.01-5 wt%.

Due to the pharmacological activity demonstrated by the compounds of formula I in several tests, various types of skin disorders can be advantageously treated with the compositions according to the present invention, such as cutaneous malignancies, primary to the skin, keratosis, ichtyosis, acne, rosacea, dermatitis (all types of eczema). Of particular concern for the present invention is the use of 7,8,9,10-tetra:hydro-thieno-[3,2-e]-pyrido-[4,3-b]-indoles and their pharmaceutically acceptable acid addition salts for the treatment of psoriasis.

All publications and patent applications cited in this specification are herein incorporated by reference as if each publication or patent application were specifically and individually indicated to be incorporated by reference.

Although the foregoing invention has been described in some detail by way of illustration and example for purposes of clarity and understanding, it will be readily apparent to those of ordinary skill in the art in light of the teachings of this invention that certain changes and modifications may be made thereto without departing from the spirit and scope of the appended claims.

The following examples will illustrate the invention.

## EXAMPLES

### Abbreviations

| | |
|---|---|
| CaM | calmodulin |
| CL | chemiluminescence |
| DMEM | Dulbecco's modified essential medium |

| | |
|---|---|
| DMSO | dimethyl sulfoxide |
| EDTA | ethylene diamine tetraacetic acid |
| EGTA | ethyleneglycol-bis (β-aminoethylether) tetraacetic acid |
| LDH | lactate dehydrogenase |
| NHEK | normal human epidermal keratinocyte |
| PBS | Dulbecco's phosphate buffered saline |
| PKC | protein kinase C |
| PMA | phorbol 12-myristate 13-acetate |
| PMNL | polymorphonuclear leukocytes |
| PS | phosphatidylserine |
| ROS | reactive oxygen species |
| W-7 | N-(6-aminohexyl)-5-chloro-1-naphthalene sulfonamide |

General

Antibiotic-antimycotic solution (P/S), fetal calf serum (FCS), and L-glutamine were from Gibco (Grand Island, USA) and all other solutions used for cell culture as well as staurosporine were delivered by Boehringer Mannheim (Mannheim, FRG). Radiotracers were obtained from NEN DuPont (Bad Homburg, FRG).

All other chemicals used for buffers and solutions as well as the reference compound W-7 were obtained from Sigma (St. Louis, USA) and were of the highest purity commercially available.

HaCaT cells are spontaneously immortalized aneuploid human keratinocytes prepared according to J. Cell. Biol. (1988), 106: 761-771. They can be substituted, however, by $SVK_{14}$ cells [Taylor-Papadimitriou et al. (1982), J. Cell Differentiat. 11: 169-180] or freshly isolated human keratinocytes [Rheinwald and Green (1975), Cell 6: 331-344].

The HaCaT cells (or the other cells) were routinely grown in Dulbecco's modified essential medium (DMEM) supplemented with 1% antibiotic-antimycotic solution, 4 mM L-glutamine and 10% fetal calf serum in culture flasks (T 175; Falcon Labware, Oxuard, CA, USA). Proliferation tests were carried out with passages 90-95 (for HaCaT-cells) and passages 3-6 (for NHEK's) in 24 well plates (Greiner, Solingen, FRG) as described below. All incubations were performed at 37°C in a humidified atmosphere with 10% $CO_2$. Test compounds were dissolved in 10% DMSO and diluted to a final medium concentration of 0.5% DMSO. This solvent concentration did not affect cell proliferation.

## Example 1

## Anti-proliferative effect of tiflucarbine on HaCaT cells

Proliferation of HaCaT cells was assayed by three different parameters: incorporation of [$^3$H]-thymidine, incorporation of [$^{14}$C]-amino acids and the increase of protein content of attached cells. HaCaT cells were seeded in supplemented DMEM (40,000 cells per culture well) and incubated for 24 h. The medium was then replaced by a medium also containing the test compounds at the concentrations indicated. Following a further 48 h incubation period, culture plates were washed twice with DMEM. Then pulse labelling was carried out by addition of 500 µl pure DMEM containing [$^3$H]-thymidine (0.5 µCi) and [$^{14}$C]-amino acids (0.25 µCi) per well and incubation was continued for 2 h. Thereafter, the medium was removed and cells were washed once with Dulbecco's phosphate buffered saline (PBS) and again with PBS containing 0.2% EDTA. Trypsin-EDTA (0.05%, 0.02%; 0.5 ml per well) was added and cells were incubated for 45 min to detach them completely from the plate. Trypsinization was stopped by addition of 0.5 ml of ice cold trichloroacetic acid (10%). After incubation for 12 h at 4°C, the cells were harvested on a glass fiber filter (G-7; Whatman, Maidstone, Kent, UK). Thereafter, the filters were treated with 0.5 ml PROTOSOL®-solution (NEN, Bad Homburg, FRG) at 60°C for 1 h and radioactivity was assessed by liquid scintillation counting. The total incorporation of radiotracers in the absence of test compounds was taken as 0% inhibition.

For determination of total protein content, cells were washed twice with methanol. Culture plates were then dried at room temperature and 1.5 ml Na0H (0.25 N) were added to each well, which was then carefully covered with PARAFILM®-foil. Plates were shaken for 16 h at room temperature. Concentration of protein was determined spectrophotometrically using bovine serum albumin as a standard. The total concentration of protein in the absence of test compounds was taken as 0% inhibition.

Proliferation of human keratinocytes, as assessed by incorporation of [$^3$H]-thymidine and [$^{14}$C]-amino acids and also by the increase of total protein content, was inhibited by tiflucarbine in a dose-dependent

manner (see Figures 1 and 2). The Figures show the mean values ± SD from four independent experiments each performed in quadruplicate (missing error bars are smaller than the symbols). All parameters measured were inhibited with nearly the same potency ($IC_{50}$ values: 10-17 µM).

**Example 2**

**Anti-proliferative effects of tiflucarbine and staurosporine on $SVK_{14}$ cells and on Normal Human Epidermal Keratinocytes**

Normal human adult epidermal keratinocytes (NHEK) were cultured using 3T3-feeder cells, in a mixture of DMEM and Ham's F12 (3:1) supplemented with 5% Foetal Calf Serum (FCS), 1 µM hydrocortisone, 1 µM isoproterenol and 10 ng EGF per ml [Rheinwald and Green (1975) Cell, 6: 331). SV40-transformed keratinocytes ($SVK_{14}$) [Taylor-Papadimitriou et al. (1982) Cell Differ., 11: 169] were cultured in the same medium, without EGF. Both cell types were grown at 37° C in a humidified atmosphere containing 7.5% $CO_2$.

On day 0, 5,000 cells in 100 µl were plated per well in a 96-well cluster plate (Greiner), either in the medium as stated above, or in MCDB153 medium (Clonetics) under low calcium conditions. On day 1 100 µl medium containing increasing concentrations of test agents were added to each well. On day 4, 50 µl of 0.05% neutral red (Gurr) solution in 0.9% NaCl was added per well and the cells were reincubated for 3 h at 37° C. After washing with PBS, the cells were lyzed by addition of 100 µl of 50% ethanol in 0.05 M $NaH_2PO_4$ per well. After vigorous shaking, the optical extinction was measured at 550 nm. The extinction is a measure for the number of viable cells, attached to the bottom of the culture plates [Borenfreund and Puerner (1984) J. Tissue Cult. Meth., 9: 7). All determinations were performed in octuple. $IC_{50}$-values (the concentration inhibiting cell numbers by 50%) were determined (µM).

Table 1

| Effect of PKC inhibitors on keratinocyte proliferation $IC_{50}$ values (µM) | | | | |
|---|---|---|---|---|
| Cell | NHEK | | $SVK_{14}$ | |
| $Ca^{2+}$ concentration | low | normal | low | normal |
| Staurosporine | 0.00025 | 0.0004 | 0.003 | 0.06 |
| Tiflucarbine | 10.0 | 10.0 | 7.5 | 12.5 |

In Table 1 it can be seen, that tiflucarbine inhibited proliferation of normal human keratinocytes and $SVK_{14}$ cells, with about the same potency as HaCaT cells. There was no difference between cells grown under low (0.06 mM) or normal (1.6 mM) calcium conditions, as was the case with the (very potent) reference compound staurosporine.

**Example 3**

**Effects of tiflucarbine and other compounds on ROS-release**

Human PMNL were isolated from fresh blood (anticoagulated with sodium citrate) by erythrocyte sedimentation with 1% dextran (Pharmacia, Uppsala, Sweden) centrifugation of the resulting supernatant, followed by hypoosmotic lysis to remove red blood cells. Washed cells were adjusted to 2 x $10^6$ cells/ml in PBS containing fetal calf serum (2%; v/v). Test compounds dissolved in DMSO were diluted with the assay buffer to a final DMSO concentration of 0.5% (v/v).

ROS generation from human PMNL (2 x $10^5$ cells in 500 µl final volume) was recorded by lucigenin-amplified chemiluminescence (CL) as reported in detail in Biochem. Pharmacol. (1987), 36: 1125-1132.

Peak CL determined the maximal cell activity 16 min after addition of 25 µl complement-opsonized zymosan (ZyC3b; 0.8 mg/ml) or PMA (2 x $10^{-7}$ M). Then the test compounds (100 µl) were added with subsequent recording for another 16 min period. Spontaneous CL was determined for a 16 min period after 30 min preincubation with the test compounds started by the addition of lucigenin. Induced CL was subsequently recorded for another 24 min after addition of the appropriate stimulus to the same test tubes. Results were expressed as percent of inhibition or stimulation of the controls (mean of two integrals) in a single experiment. $IC_{50}$-values and limit of confidence (95%) were calculated by regression analysis.

Tiflucarbine also inhibited the ROS generation from isolated human PMNL. There was no difference in the potency of tiflucarbine to inhibit ROS production in unstimulated cells and cells stimulated with opsonized zymosan (Figure 3) or PMA (Figure 4). Both Figures show the mean values ± SD from three independent experiments each performed in triplicate (missing error bars are smaller than the symbols). The effects were brought about with $IC_{50}$ values of 7 µM. However, with both stimuli inhibition of peak chemiluminescence to 50% was observed at 2-3 fold higher concentrations.

Table 2 shows data obtained with several reference compounds after stimulation with opsonized zymosan.

Table 2

| Compound | Spontaneous | CL Peak CL | Induced CL |
|---|---|---|---|
| Staurosporine | 0.015 | 0.016 | 0.023 |
| W-7 | 6.3 | 10.0 | 6.3 |
| Trifluoroperazine | 4.5 | 43.8 | 22.7 |
| Thioridazine | 2.4 | 10.6 | 6.4 |
| Chlorpromazine | 4.9 | 21.8 | 7.6 |
| Promethazine | 23.6 | 62.3 | 31.4 |
| Tiflucarbine | 7.2 | 14.0 | 7.4 |

**Example 4**

**Cytotoxicity of tiflucarbine and other PKC-inhibitors**

HaCaT cells were grown as described.

Cytotoxicity was assessed by measurements of the content of intracellular lactate dehydrogenase (LDH) activity released into the culture medium during 48 h after onset of drug exposure. Possible drug induced detachment of the HaCaT cells from the plate was assessed by collection of the medium from the wells and centrifugation (80 x g, 10 min.) The resulting pellet was suspended in 10 µl medium and cells were counted in a haemocytometer.

Nonspecific cytotoxicity of the test compounds on PMHL was assessed by trypane blue exclusion test.

Table 3

| Compound | LDH release (µM) | Trypane blue (µM) |
|---|---|---|
| Staurosporine | 10 | 3 |
| W-7 | 100 | 15 |
| Trifluoroperazine | 30 | 100 |
| Thioridazine | 30 | 30 |
| Chlorpromazine | 30 | 30 |
| Promethazine | 100 | 100 |
| Tiflucarbine | 300 | 300 |

As can be seen in Table 3, tiflucarbine is toxic above 300 µM only. Other PKC-inhibitors are much more toxic.

**Example 5**

**Effect of tiflucarbine and staurosporine on keratinocyte differentiation**

Cornified envelope formation:

Normal human keratinocytes were grown to confluence at low calcium concentration in the presence of 5% FCS, 1 µM isoproterenol and 10 ng EGF per ml. They were first exposed for 4 h to increasing

concentrations of test compounds and subsequently reincubated for 48 h in medium containing normal calcium concentration and the same additives, test compounds and [$^3$H]-leucine (1 µCi/ml). Thereafter, both spontaneous and ionophore-induced cornified envelope (CE) formation was determined [King et al. (1986) Exp. Cell. Res., 167: 252].

It can be seen in Figure 5, that tiflucarbine has no effect on spontaneous CE formation, while staurosporine has a marked effect. No drug tested had effect on induced CE formation (Figure 6).

Keratin expression:

HaCaT cells were cultured under standard conditions. 24 h after plating (2.5 x $10^6$ cells) the medium was replaced by medium containing [$^{14}$C]-amino-acids (0.5 µCi/ml) and the test compounds. After 72 h, cultivation was completed, keratinocytes were harvested using a rubber policeman and homogenized in 10 mM TRIS/Cl (pH 7.2) containing 1.5 M KCl, 0.5% Triton X 100, 5 mM EDTA and 0.4 mM PMSF (phenyl methyl sulfonyl fluoride) using a Polytron cell disrupter. After centrifugation (3500 x g, 10 min) the pellet was resuspended in 10 mM TRIS/Cl (pH 7.2) containing 5 mM EDTA and 0.4 mM PMSF and subjected to another centrifugation step (3500 x g, 10 min). The resulting pellet was dissolved in either 63 mM TRIS/Cl (pH 7.4) containing sodium dodecyl sulphate (SDS) for one-dimensional gel electrophoresis or in 9 M urea for two-dimensional gel elctrophoresis. One-dimensional sodium dodecyl sulphate-polyacrylamide gel electrophoresis (SDS-PAGE) was conducted [Leammli (1970) Nature, 227: 680] using a 12% separating and a 3.5% stacking gel. Two-dimensional electrophoresis was performed [O'Farrel et al. (1987) Cell, 12: 1133]. Separation of keratins was achieved by non-equilibrium pH gradient electrophoresis (NEpHGE) using Servalyt 2-11 (Serva, Heidelberg, FRG) in the first dimension and by 12% SDS-PAGE in the second dimension.

Gels were stained with Coomassie brilliant blue R (Bio Rad, München, FRG), dried and exposed to X-ray films for 7 to 10 days at -70°C. The autoradiographs were evaluated using a density scanner. The areas of the individual peaks were calculated and related to the molecular weight of the respective keratin. Synthesis of the various keratins was expressed in percent of total keratin synthesis. Figure 7 shows the two-dimensional gel patterns, Figure 8 shows the relative amounts of the different keratins.

## Example 6

### Effects of tiflucarbine and staurosporine on keratinocyte morphology

HaCaT cells were cultured under standard conditions. 24 h after plating (2.5 x $10^6$ cells) the medium was replaced by medium containing the test compounds. During 72 hours cell morphology was frequently monitored using a phase contrast photomicroscope (Olympus IMT-2, Tokyo, Japan).

As can be seen in Figure 9, staurosporine (30 nM) has effects on morphology. Tiflucarbine (10 µM) had no effect (not shown).

## Example 7

### Effect of tiflucarbine and polymyxin B on PKC from the central nervous system

Phosphorylation of synaptosomal plasma membranes (SPM):

Subcellular fractionation and phosphorylation assay were performed according to Kristjansson et al., (1982). The phosphorylation reaction mixture (final volume 25 µl) contained 10 µg SPM protein, 7.5 µM ATP and 1 µCi [gamma $^{32}$P]ATP in 10 mM TRIS/Cl, 10 mM $MgCl_2$, 10 mM $CaCl_2$, pH 7.4. After a preincubation at 30°C for 5 min, the phosphorylation reaction was started by adding the ATP. The reaction was stopped after 15 sec. All compounds to be tested were present at the start of the preincubation. Proteins were separated by 11% SDS-PAGE, protein phosphorylation was determined by autoradiography of the gel and quantified by densitometric scanning of the autoradiogram.

Phosphorylation of B-50 and the 50 kDa protein, which was only phosphorylated after addition of CaM, was measured in the presence of exogenous CaM (3 units/assay) and quantified.

Unexpectedly, tiflucarbine did not inhibit B-50 phosphorylation, whereas the CaM-dependent phosphorylation of the 50 kDa protein was dose-dependently inhibited with a high potency of the drug ($IC_{50}$-value smaller than 1 µM; see Figure 10). In order to exclude a possible interference of CaM with the inhibitory action of tiflucarbine on PKC, the effect of the drug was assessed without addition of CaM to the

assay. Under this condition, tiflucarbine did not display any inhibitory effect on B-50 phosphorylation as well. The phosphorylation of B-50 (% of control) was 95 ± 11, 92 ± 9, 110 ± 15 and 82 ± 14 for tiflucarbine concentrations of l mM, 100 $\mu$M, 10 $\mu$M and 1 $\mu$M, respectively.

For control, the dual PKC/CaM inhibitor, polymyxin B, was used in the same assay. Polymyxin B dose-dependently inhibited the phosphorylation of B-50 and the 50 kDa protein (see Figure 11) in the presence of added CaM. Without exogenous CaM inhibition of B-50 phosphorylation of 18 ± 8, 82 ± 11, 103 ± 9 and 95 ± 8% of control was achieved under polymyxin B concentrations of 100, 10, 1 and 0.1 $\mu$M, respectively.

Noradrenaline release from permeated synaptosomes:

Highly purified synaptosomes were prepared according to Dunkley and Robinson (1987). The synaptosomes were diluted with 3 ml buffer A (123 mM NaCl, 5 mM KCl, 2 mM $CaCl_2$, mM $MgCl_2$, 1.15 mM $NaH_2PO_4$, 5.6 mM glucose, 20 mM PIPES pH 6.8, gassed with $O_2$:$CO_2$, 95:5) and incubated for 15 min at 37°C. Then 2.5 $\mu$Ci noradrenalin (NA) was added, incubation was continued for 15 min and the synaptosomes were centrifuged for 20 min at 600 x g. The resulting pellet was washed twice in buffer A without $CaCl_2$ and resuspended (1 mg protein/ml).

For measurement of NA release, prelabeled synaptosomes were incubated at 37°C in buffer A containing $Na_2$ATP and EGTA-buffered $Ca^{2+}$ in concentrations as indicated (final volume 120 $\mu$l). The release reaction was started by adding synaptosomes (20 $\mu$g protein) to the reaction mixture and terminated after 5 min by 30 sec centrifugation at 10,000 x g. The supernatant (90 $\mu$l) was collected and counted in a Packard Model 2,000 CA liquid scintillation counter with Picofluor as a scintillation cocktail. NA release was expressed as % of total [$^3$H]-NA incorporated. Total NA incorporation was determined by measuring [$^3$H]-incorporation in 20 $\mu$g prelabeled washed synaptosomes.

As can be seen in Figure 12, tiflucarbine, tested in two independent experiments in concentrations ranging from 100 $\mu$M to 1 $\mu$M, did not display any significant effects on the noradrenaline release. Both, the release under low (10 nM) and high (10 $\mu$M) $Ca^{2+}$ remained in the range of the control values. In contrast, 100 $\mu$M of polymyxin B markedly inhibited this cellular response to 16.5 ± 1.03% of total [$^3$H]-noradrenaline under low and 19.4 ± 2.38% of total [$^3$H]-noradrenaline under high $Ca^{2+}$ conditions.

**Example 8**

**Effects of topically applied tiflucarbine, cyclosporine A and sphingosine on TPA-induced edema and hyperproliferation in mouse skin**

Hairless mice (hr/hr; male) were treated on their backs with 10 $\mu$l of a 0.01 wt% solution of 12-O-tetradecanoylphorbol-13-acetate (TPA) in a 80/20 mixture of acetone/water, further containing the test substances cyclosporin A, sphingosine or tiflucarbine. Per mouse, 4 application sites were used: one application site was treated with only TPA (control) and one with only vehicle (blanco), two with experimental formulations, according to a roulation scheme. 6 mice were treated per experiment.

Skin fold thickness was determined before treatment (t=0) and 6 and 24 h after treatment, as a measure of edema. After 72 h the epidermis (15 mm punch biopsy) was separated with 2 M KBr and dissolved in 0.1 M NaOH for determination of the amount of protein, as a measure of hyperproliferation.

In one experiment tiflucarbine was compared with cyclosporin A, in a second experiment with sphingosine.

As can be seen in Figure 13, tiflucarbine reduces edema at 0.5, 1 and 2.5%. It reduces hyperproliferation only at 1 and 2.5% (see Figure 14).

**Example 9**

**Effects of intraperitoneally administered tiflucarbine and sphingosine on TPA-induced ear edema in mice**

Mice were injected intraperitoneally with 30, 10 or 3.3 mg/kg body weight of the test substances, sphingosine in carboxymethylcellulose (0.5%) and tiflucarbine in physiologic Saline, 10 ml/kg body weight. After 60 minutes the mire were challenged on the left ear with a 0.01 wt% solution of 12-O-tetradecanoylphorbol-13-acetate (TPA) in a 80/20 mixture of acetone/water. At 0, 3, 6 and 24 h after challenge, ear thickness was determined.

As can be seen in Figure 15, tiflucarbine reduced ear edema at 30 mg/kg, 3 h after challenge.

Sphingosine was more potent in this respect.

**Example 10**

**Tiflucarbine topical nail lotion (0.5%)**

In a beaker 40 g of methyl ethyl ketone, 20 g of isopropanol and 39.5 g of distilled water are mixed. To this mixture tiflucarbine is added, while stirring until a clear solution is obtained.

**Example 11**

**Tiflucarbine Scalp lotion (0.5%)**

0.35 g of KLUCEL HF® is dispersed into 49 g of water, while stirring until a clear liquid is obtained. 0.5 g of isopropyl-myristate is dissolved in 50 g of isopropanol. The second solution is mixed with the first one. 0.5 g of tiflucarbine is added to the resulting mixture, while stirring.

**Example 12**

**Tiflucarbine fatty cream (0.01%)**

190 g of white soft paraffin, 90 g of liquid paraffin, 30 g of cetostearyl alcohol and 14.75 g of polyethylene glycol-1000-monocetyl ether (CETOMACROGOL 1000®) are melted and heated until a temperature of 70-80 ° C is reached. 1 g of methylhydroxy-benzoate is added to 150 g of water (de-ionized). While stirring, this solution is heated in a suitable ointment mixer to 70-80 ° C. To this solution the prepared liquid fatty composition is added at a temperature of 70-80 ° C. After vigorous mixing, under reduced pressure, the mass is gently stirred until cool. 0.25 g of CETOMACROGOL 1000 is dissolved in 25 g of water. 0.05 g of tiflucarbine is added thereto while stirring. After adding the resulting suspension to the ready cream, the mass is gently stirred for 5 min, under reduced pressure.

## Claims

1. Use of 7,8,9,10-tetra:hydro-thieno-[3,2-e]-pyrido-[4,3-b]-indoles of formula I

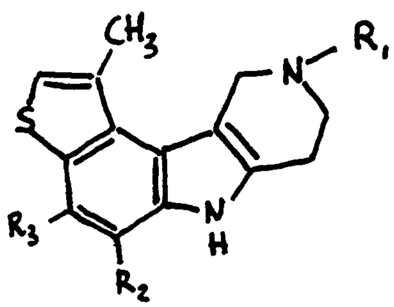

I

wherein $R_1$ is hydrogen or $C_1$-$C_4$-alkyl;
$R_2$ and $R_3$ may be the same or different, and are selected from hydrogen and/or a halogen; and the pharmaceutically acceptable acid addition salts thereof for preparing a medicament for the treatment of epidermal hyperproliferative and/or inflammatory skin diseases.

2. Use according to claim 1 of a tetra:hydro-thieno-pyrido-indole compound of formula I, wherein $R_1$ is methyl or ethyl; and $R_2$ and $R_3$ may be the same or different, and are selected from hydrogen and/or fluorine and/or chlorine; and the pharmaceutically acceptable acid addition salts thereof.

3. Use according to claim 1 of 1-methyl-9-ethyl-4-fluoro-7,8,9,10-tetra:hydro-thieno-[3,2-e]-pyrido-[4,3-b]-indole lactate.

4. Use of a compound according to any one of claims 1-3 for preparing a medicament for the treatment of

psoriasis.

**5.** A composition for the treatment of epidermal hyperproliferative and/or inflammatory skin disorders, which comprises a compound of formula I

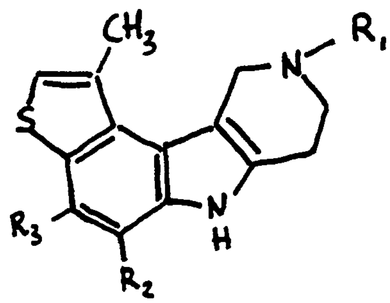

I

wherein $R_1$ is hydrogen or $C_1$-$C_4$-alkyl, preferably methyl or ethyl;
$R_2$ and $R_3$ may be the same or different, and are selected from hydrogen and/or a halogen, preferably chlorine and/or fluorine; and the pharmaceutically acceptable acid addition salts thereof as the active ingredient.

**6.** A composition for the treatment of psoriasis, which comprises a compound of formula I

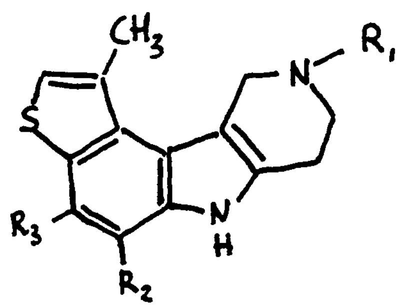

I

wherein $R_1$ is hydrogen or $C_1$-$C_4$-alkyl, preferably methyl or ethyl;
$R_2$ and $R_3$ may be the same or different, and are selected from hydrogen and/or a halogen, preferably chlorine and/or fluorine; and the pharmaceutically acceptable acid addition salts thereof as the active ingredient.

**7.** A composition according to claim 5 or 6, which comprises 0.001-20 wt% of the active ingredient, the percentage being based on the weight of the composition.

**8.** A composition according to claim 5 or 6, which comprises 0.01-5 wt% of the active ingredient, the percentage being based on the weight of the composition.

**9.** A composition according to any one of claims 5-8, characterized in that the composition is used for topical treatment.

**10.** Method of cosmetic or medical treatment of the skin, comprising the application to the body of a composition, which comprises a compound of formula I

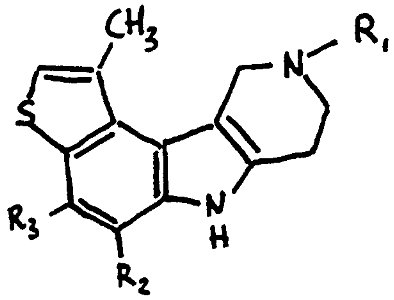

I

wherein $R_1$ is hydrogen or $C_1$-$C_4$-alkyl, preferably methyl or ethyl;
$R_2$ and $R_3$ may be the same or different, and are selected from hydrogen and/or a halogen, preferably chlorine and/or fluorine; and the pharmaceutically acceptable acid addition salts thereof as the active ingredient.

**11.** Method of cosmetic or medical treatment of the skin, comprising the topical application to the by hyperproliferation and/or inflammation afflicted areas of the skin of a composition, which comprises a compound of formula I

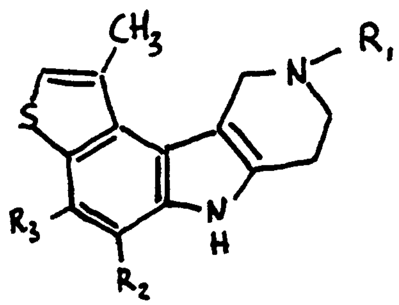

I

wherein $R_1$ is hydrogen or $C_1$-$C_4$-alkyl, preferably methyl or ethyl;
$R_2$ and $R_3$ may be the same or different, and are selected from hydrogen and/or a halogen, preferably chlorine and/or fluorine; and the pharmaceutically acceptable acid addition salts thereof as the active ingredient.

FIG. 1/15

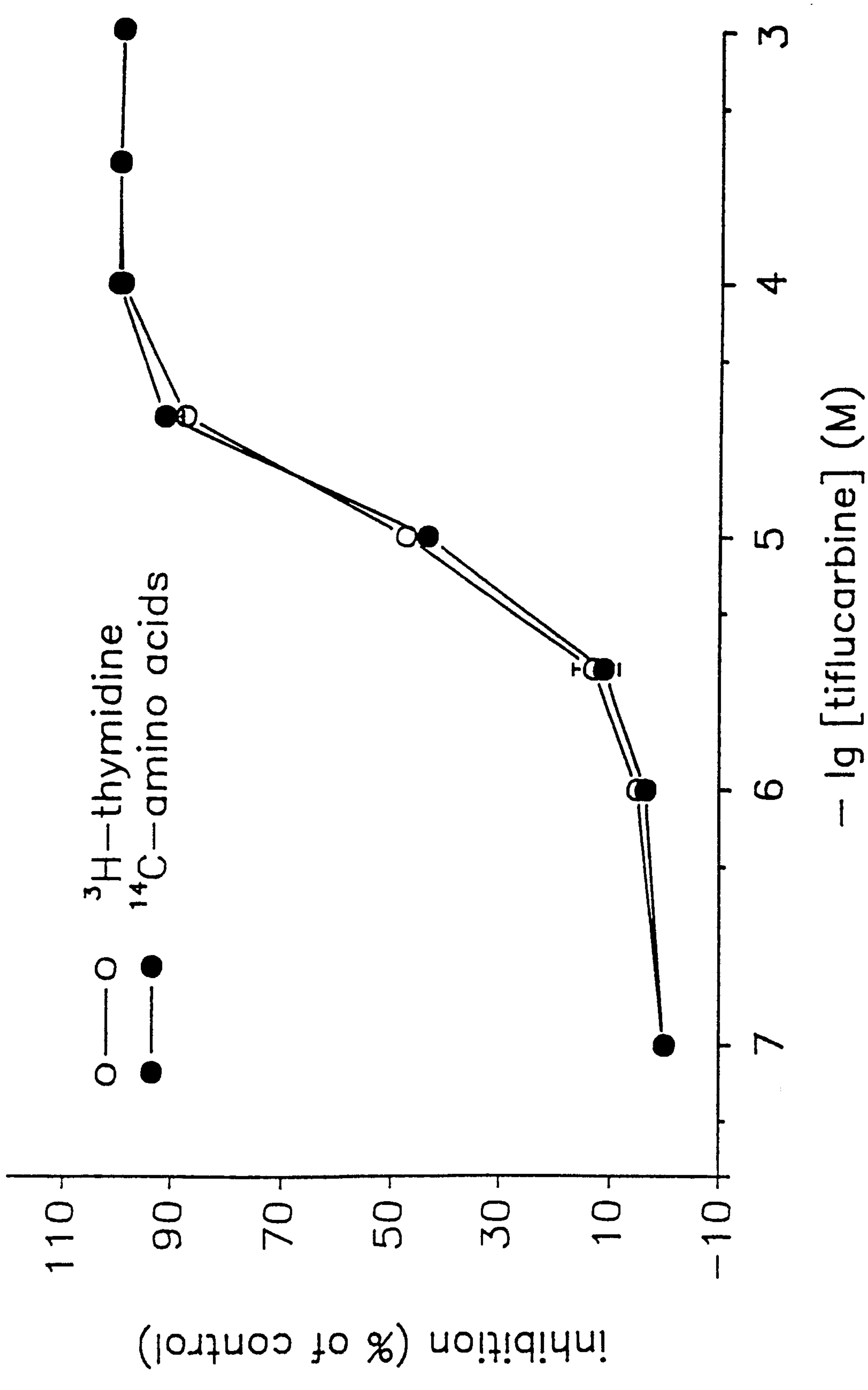

o——o $^3$H—thymidine
●——● $^{14}$C—amino acids
inhibition (% of control)
110
90
70
50
30
10
−10
7
6
5
4
3
− lg [tiflucarbine] (M)

**FIG. 2/15**

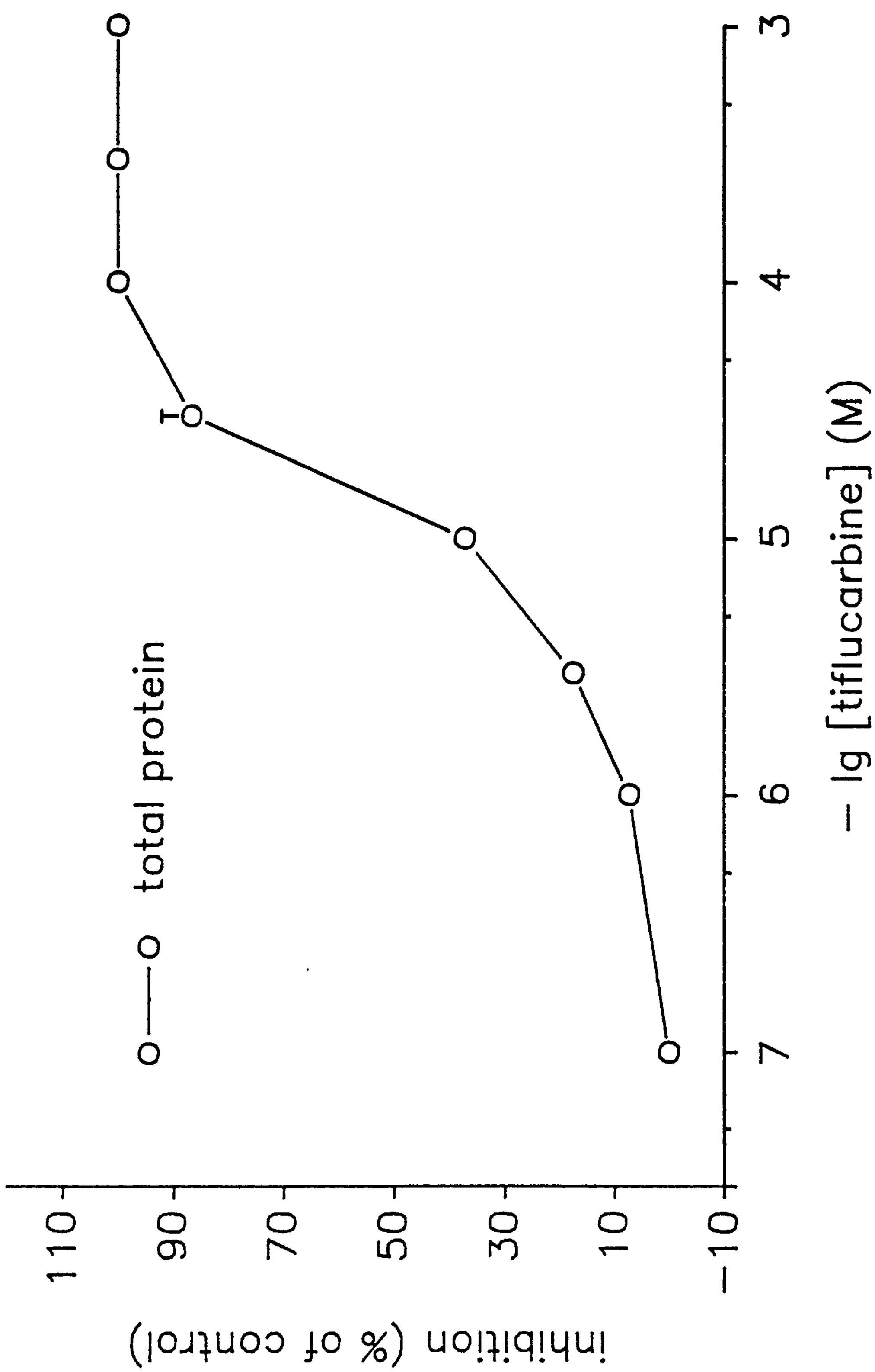

○—○ total protein
inhibition (% of control)
110
90
70
50
30
10
−10
3
4
5
6
7
− lg [tiflucarbine] (M)

FIG. 3/15

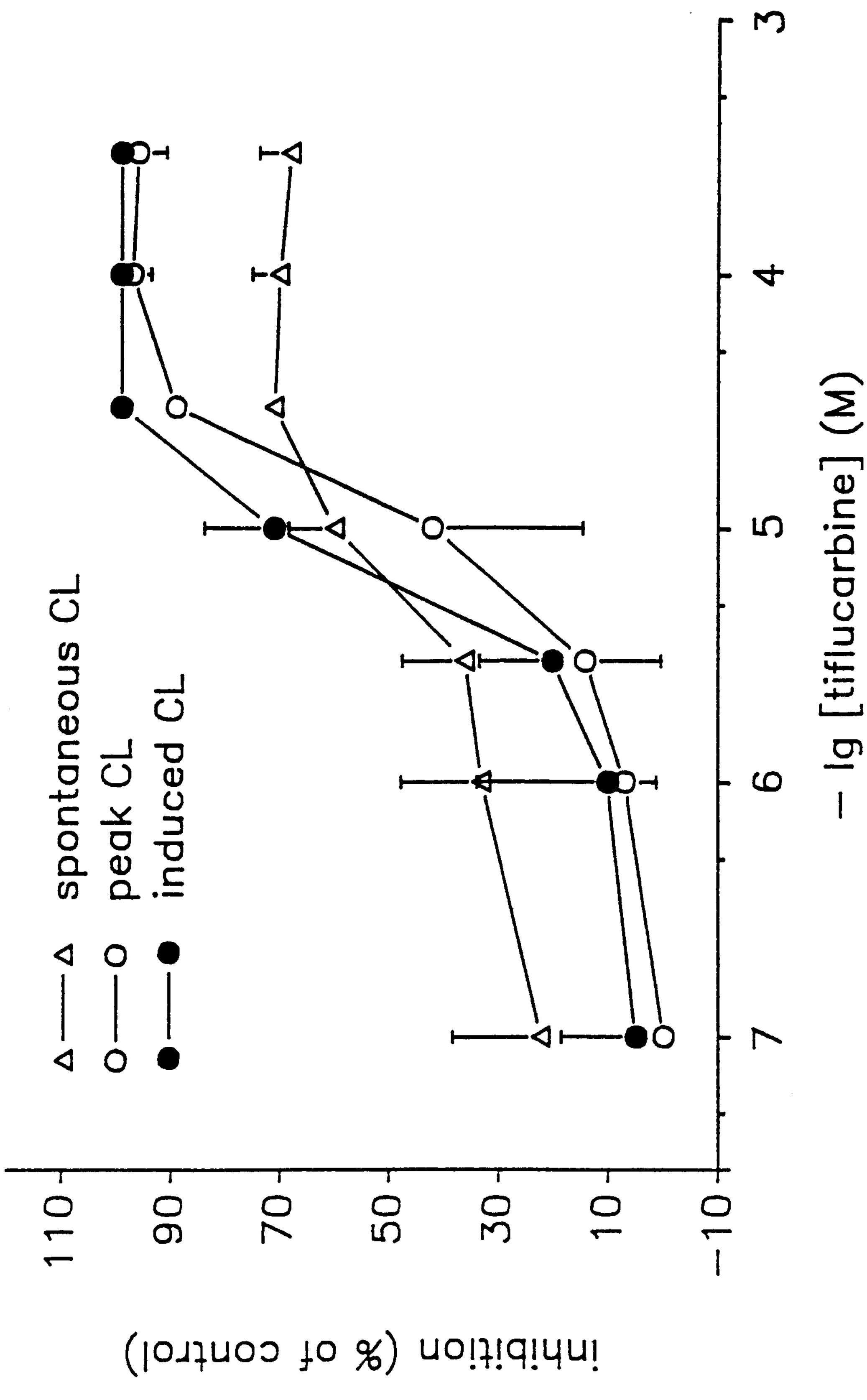
spontaneous CL
peak CL
induced CL
inhibition (% of control)
110
90
70
50
30
10
−10
7
6
5
4
3
– lg [tiflucarbine] (M)

FIG. 4/15

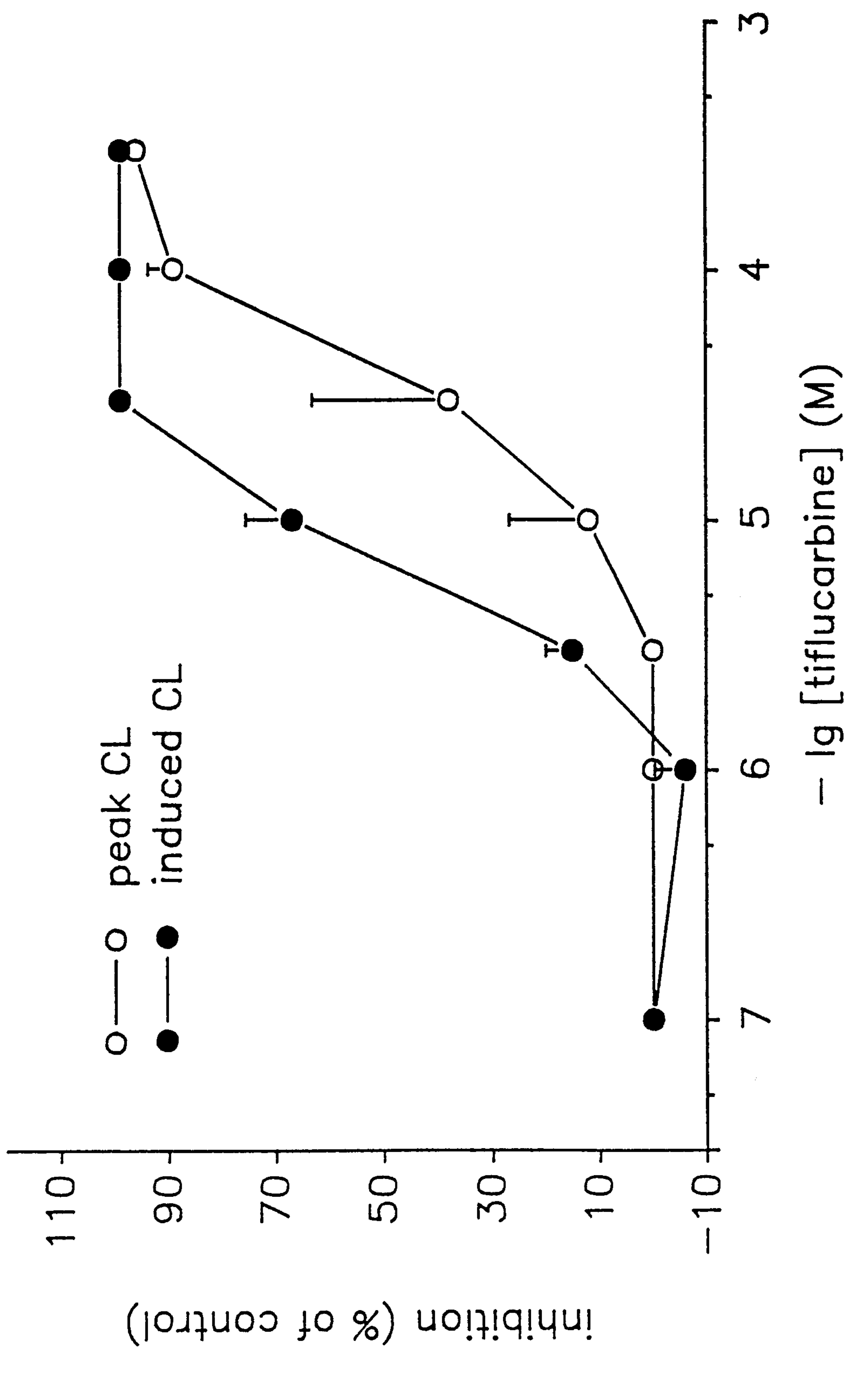
peak CL
induced CL
inhibition (% of control)
– lg [tiflucarbine] (M)
110
90
70
50
30
10
–10
7
6
5
4
3

FIG. 5/15

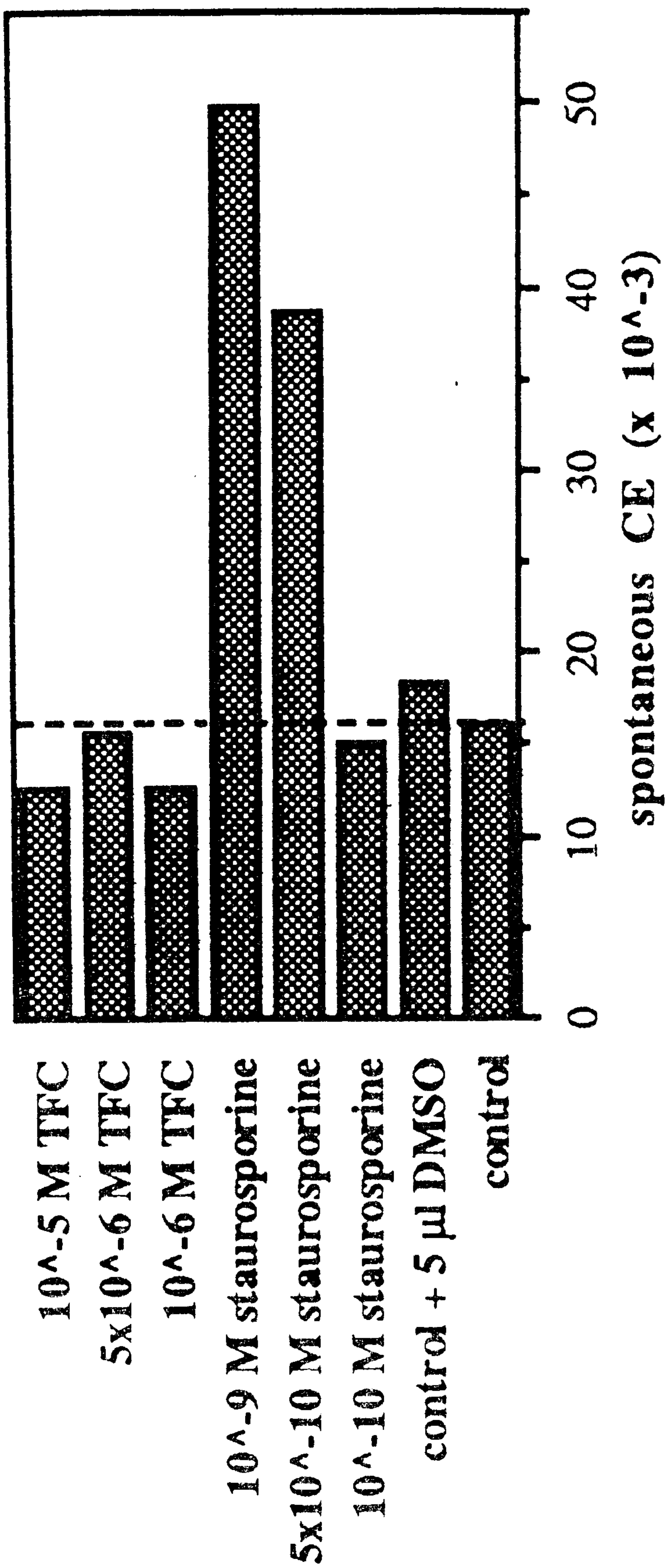
spontaneous CE (x 10^-3)
0
10
20
30
40
50
10^-5 M TFC
5x10^-6 M TFC
10^-6 M TFC
10^-9 M staurosporine
5x10^-10 M staurosporine
10^-10 M staurosporine
control + 5 µl DMSO
control

FIG. 6/15

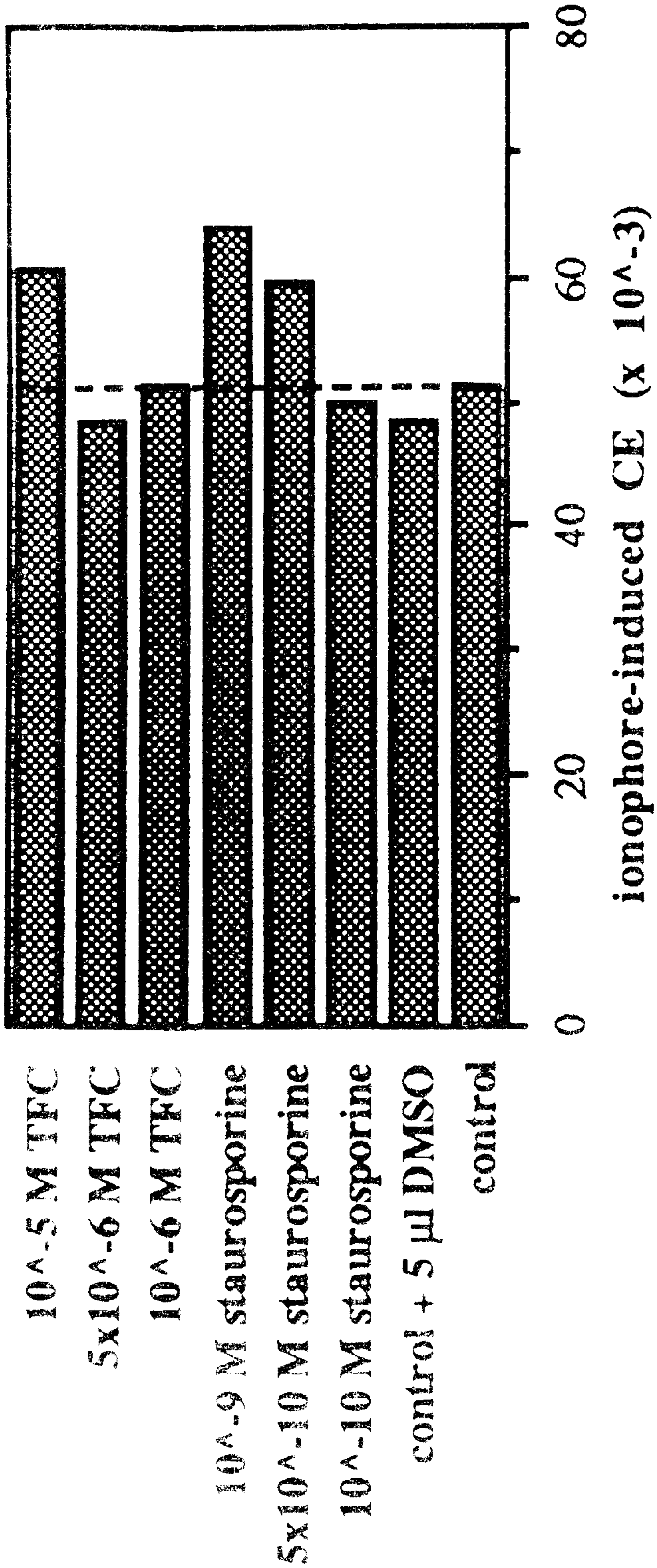
10^-5 M TFC
5x10^-6 M TFC
10^-6 M TFC
10^-9 M staurosporine
5x10^-10 M staurosporine
10^-10 M staurosporine
control + 5 µl DMSO
control
0
20
40
60
80
ionophore-induced CE (x 10^-3)

FIG. 7/15

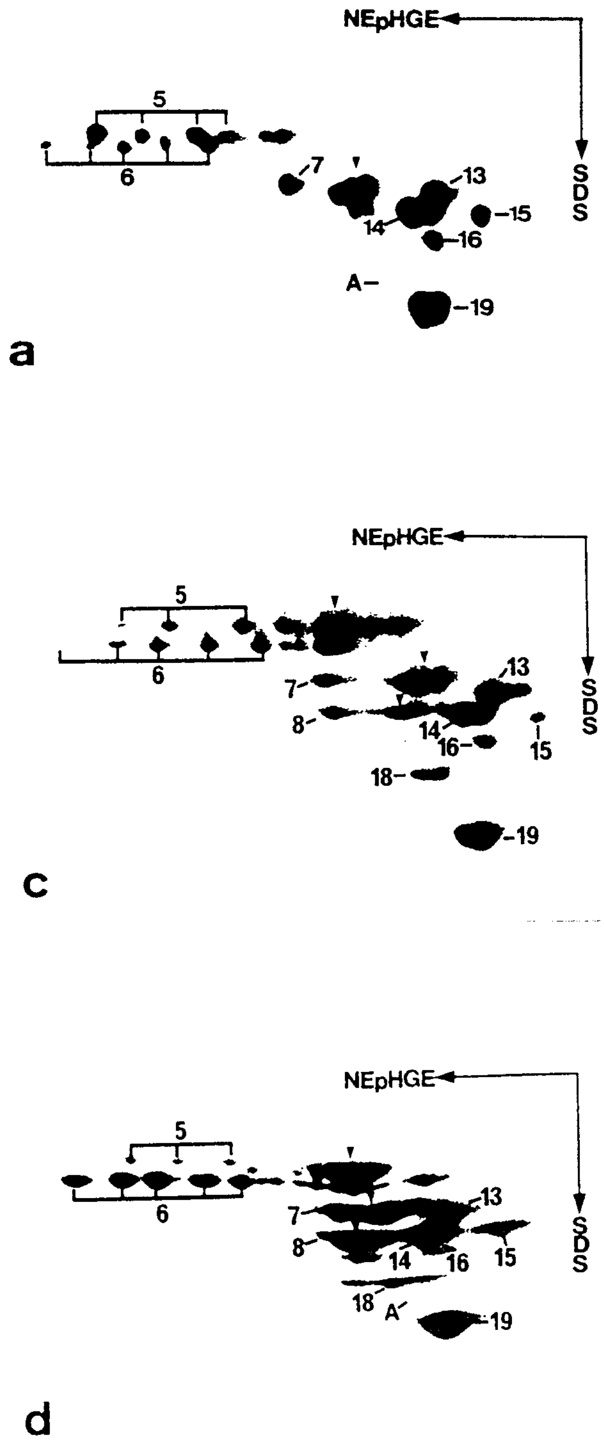
NEpHGE
SDS
5
6
7
13
14
15
16
A–
19
a
NEpHGE
SDS
5
6
7
8
13
14
15
16
18
19
c
NEpHGE
SDS
5
6
7
8
13
14
15
16
18
A
19
d

**FIG. 8/15**

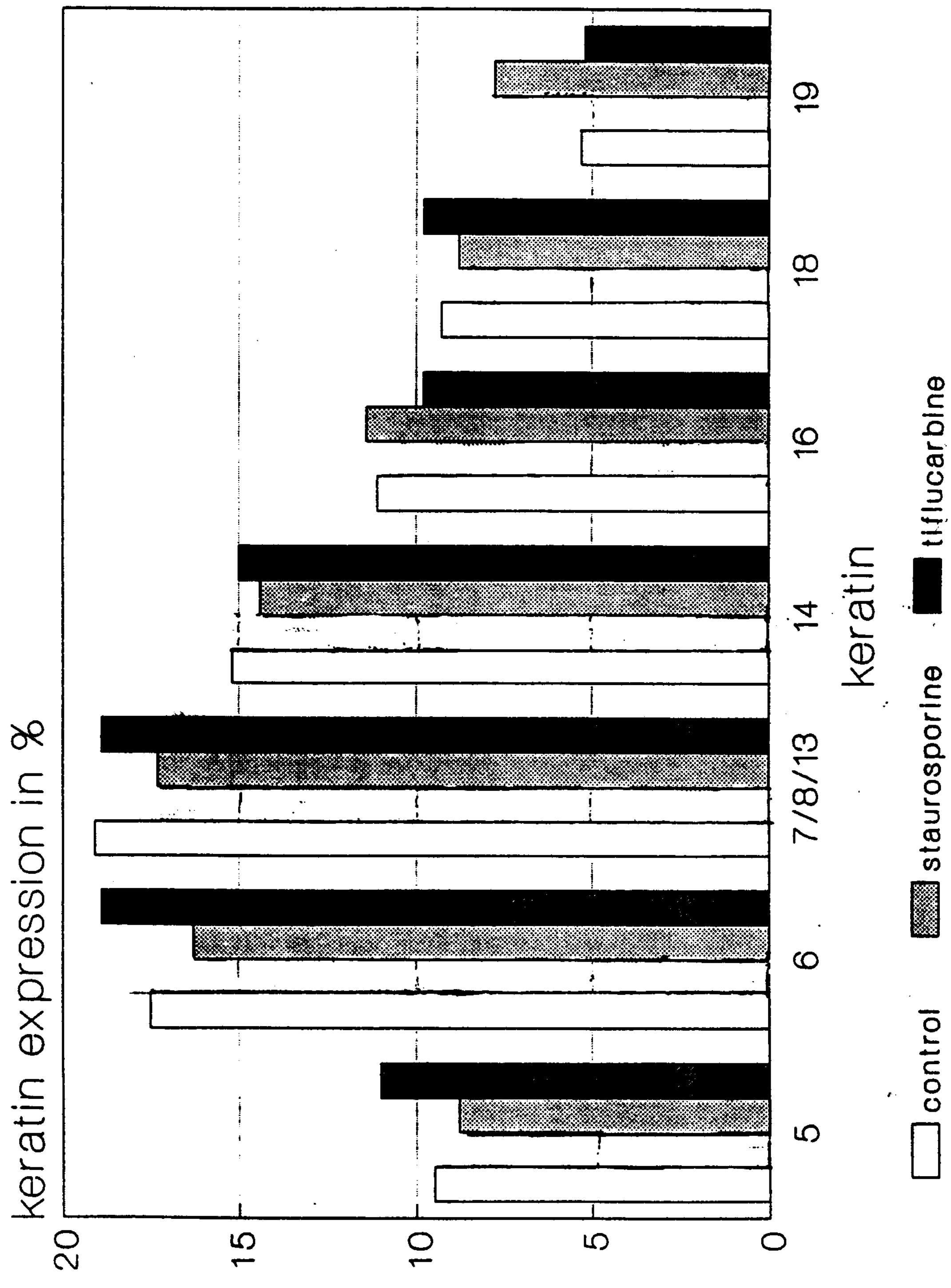
keratin expression in %
20
15
10
5
0
5
6
7/8/13
14
16
18
19
keratin
control
staurosporine
tiflucarbine

FIG. 9/15

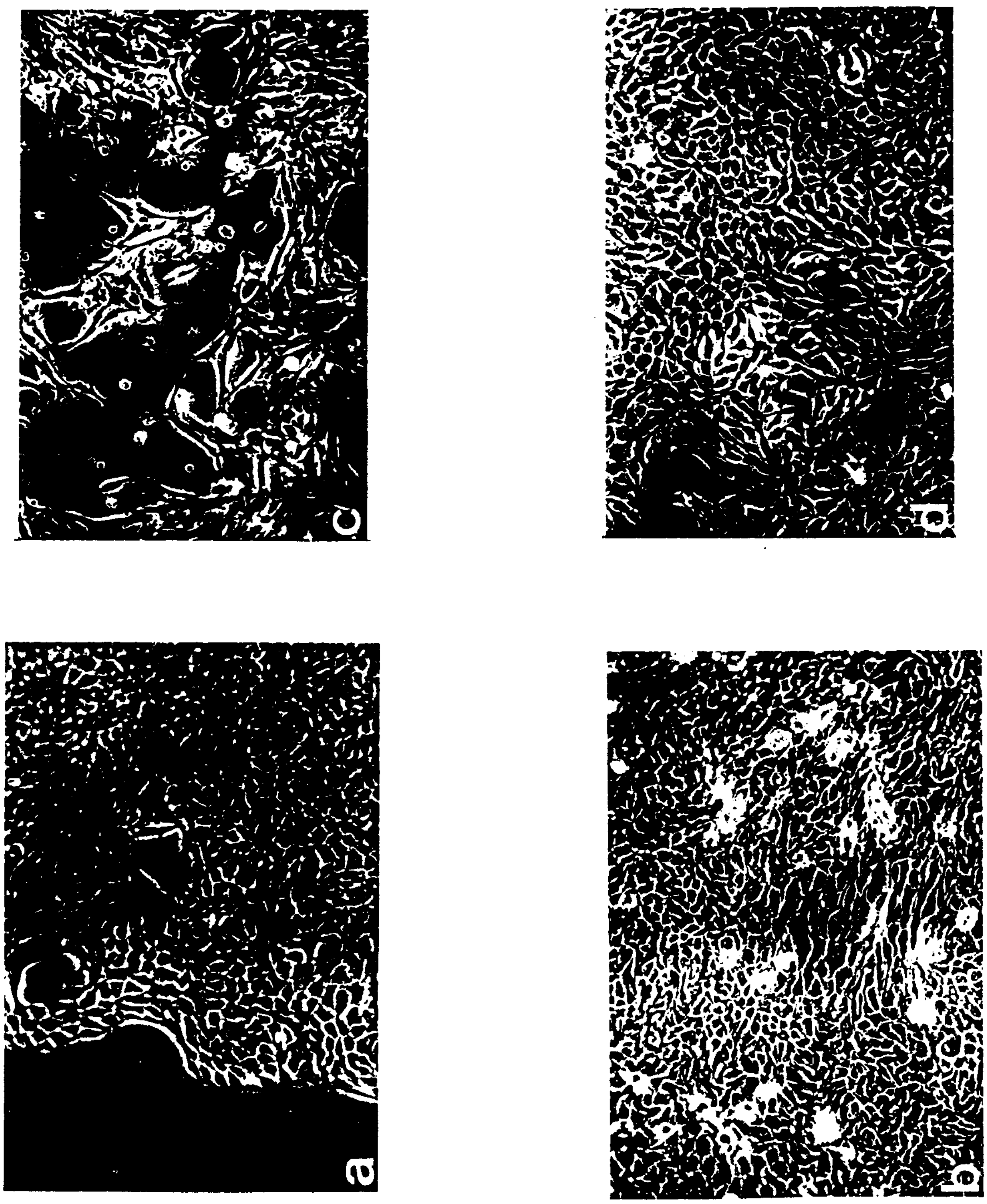

FIG. 10/15

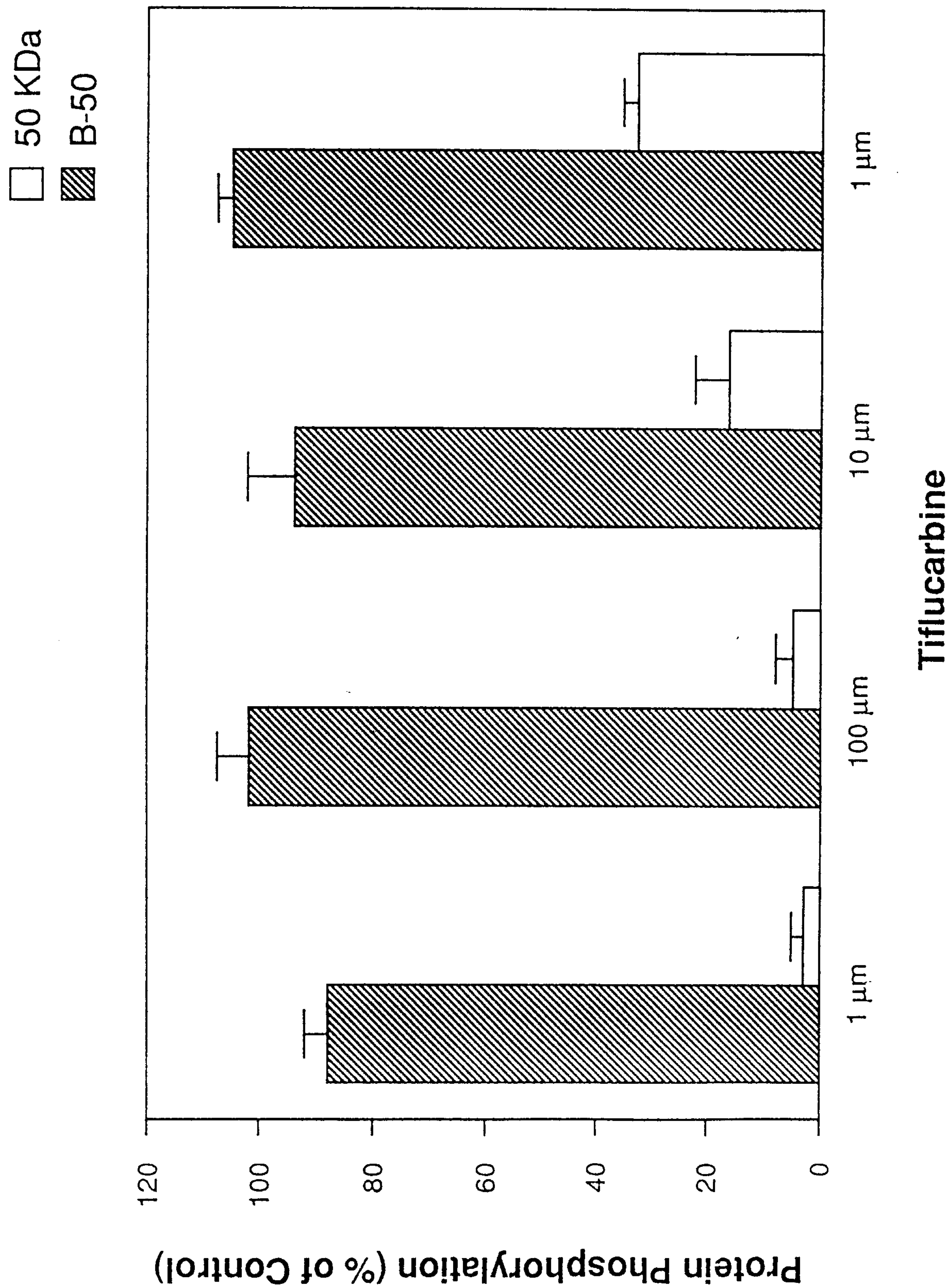
50 KDa
B-50
Protein Phosphorylation (% of Control)
0
20
40
60
80
100
120
1 µm
100 µm
10 µm
1 µm
Tiflucarbine

FIG. 11/15

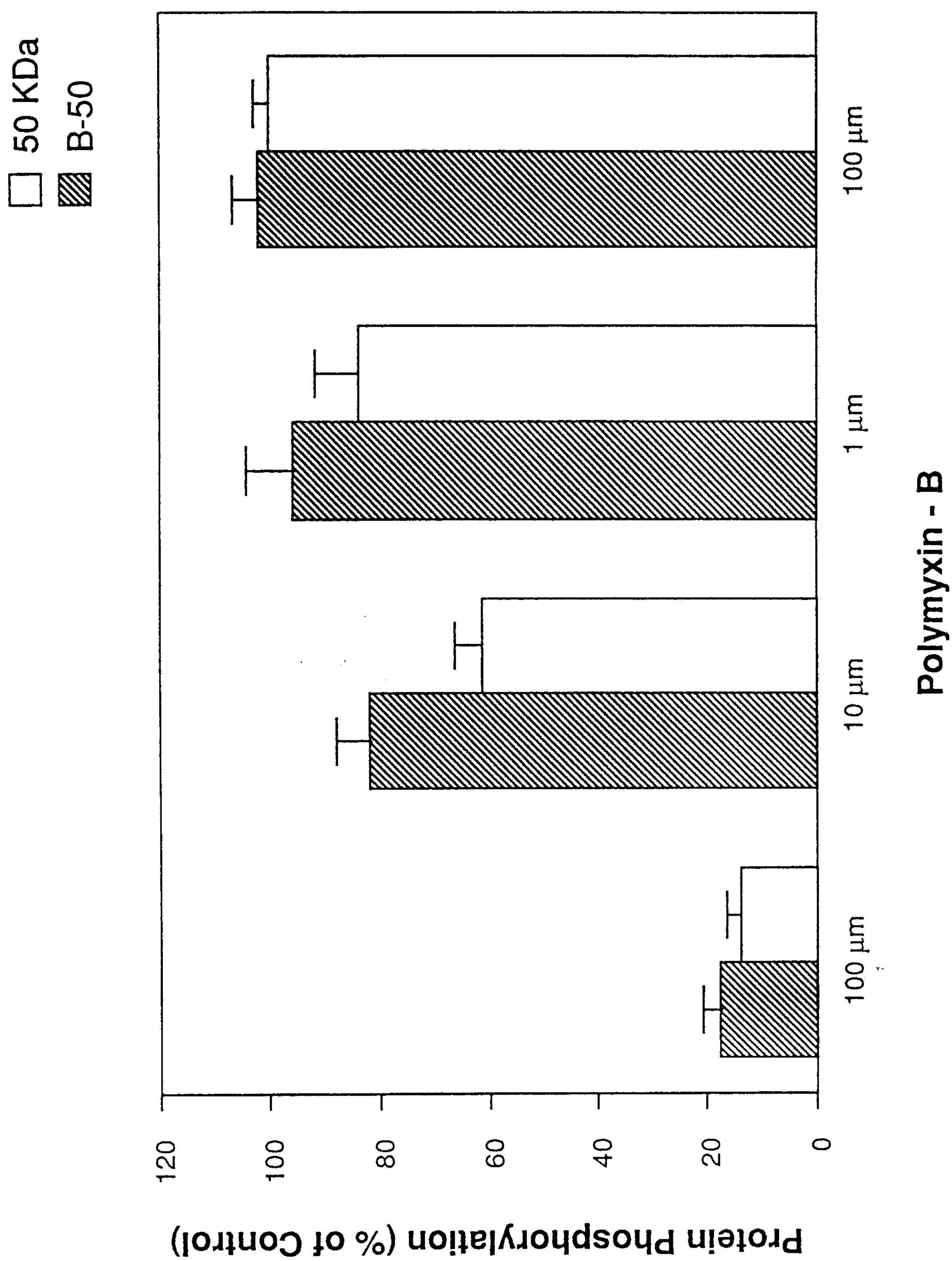
50 KDa
B-50
Protein Phosphorylation (% of Control)
0
20
40
60
80
100
120
100 µm
10 µm
1 µm
100 µm
Polymyxin - B

**FIG. 12/15**

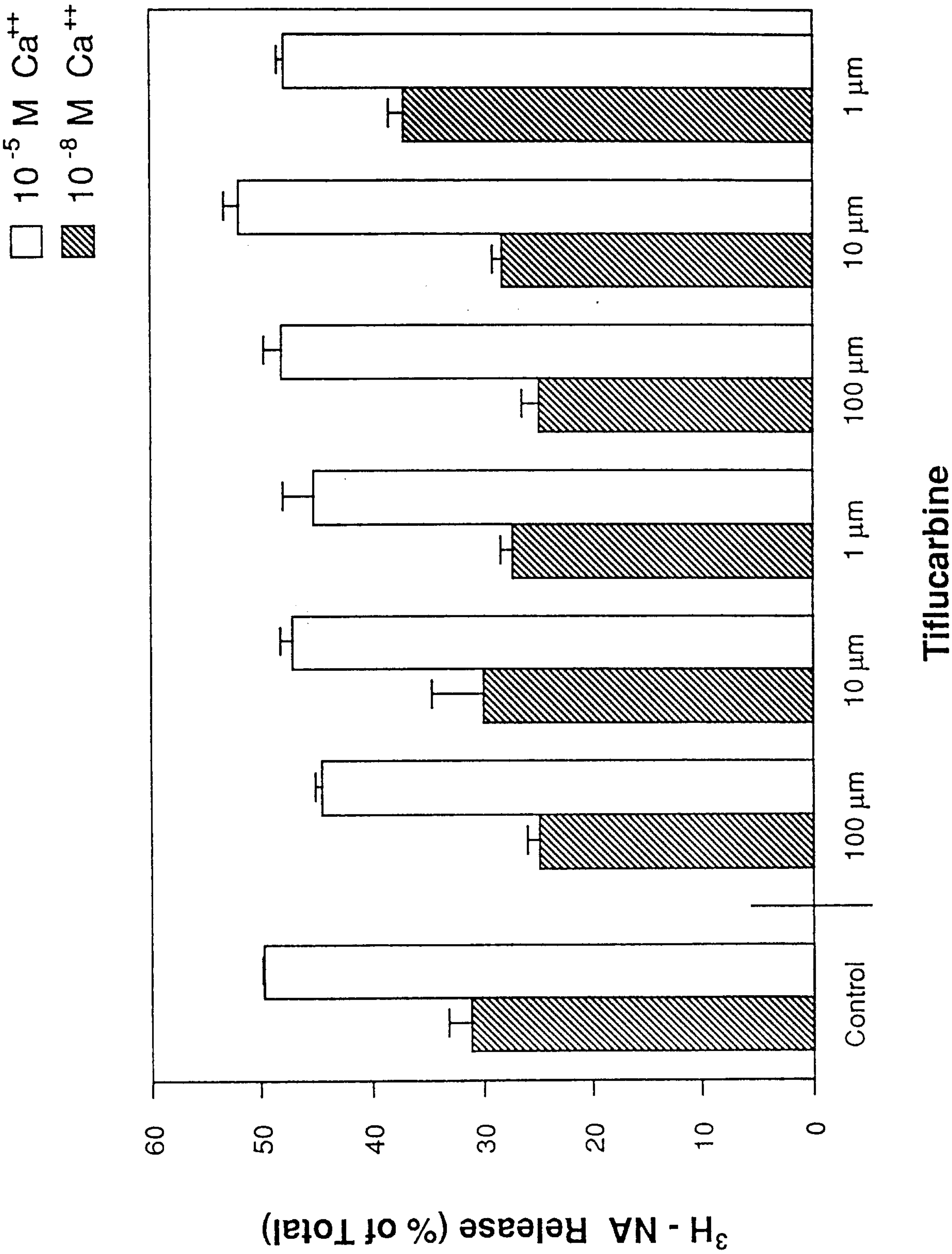
$10^{-5}$ M $Ca^{++}$
$10^{-8}$ M $Ca^{++}$
0
10
20
30
40
50
60
$^3H$ - NA Release (% of Total)
Control
100 µm
10 µm
1 µm
100 µm
10 µm
1 µm
Tiflucarbine

FIG. 13/15

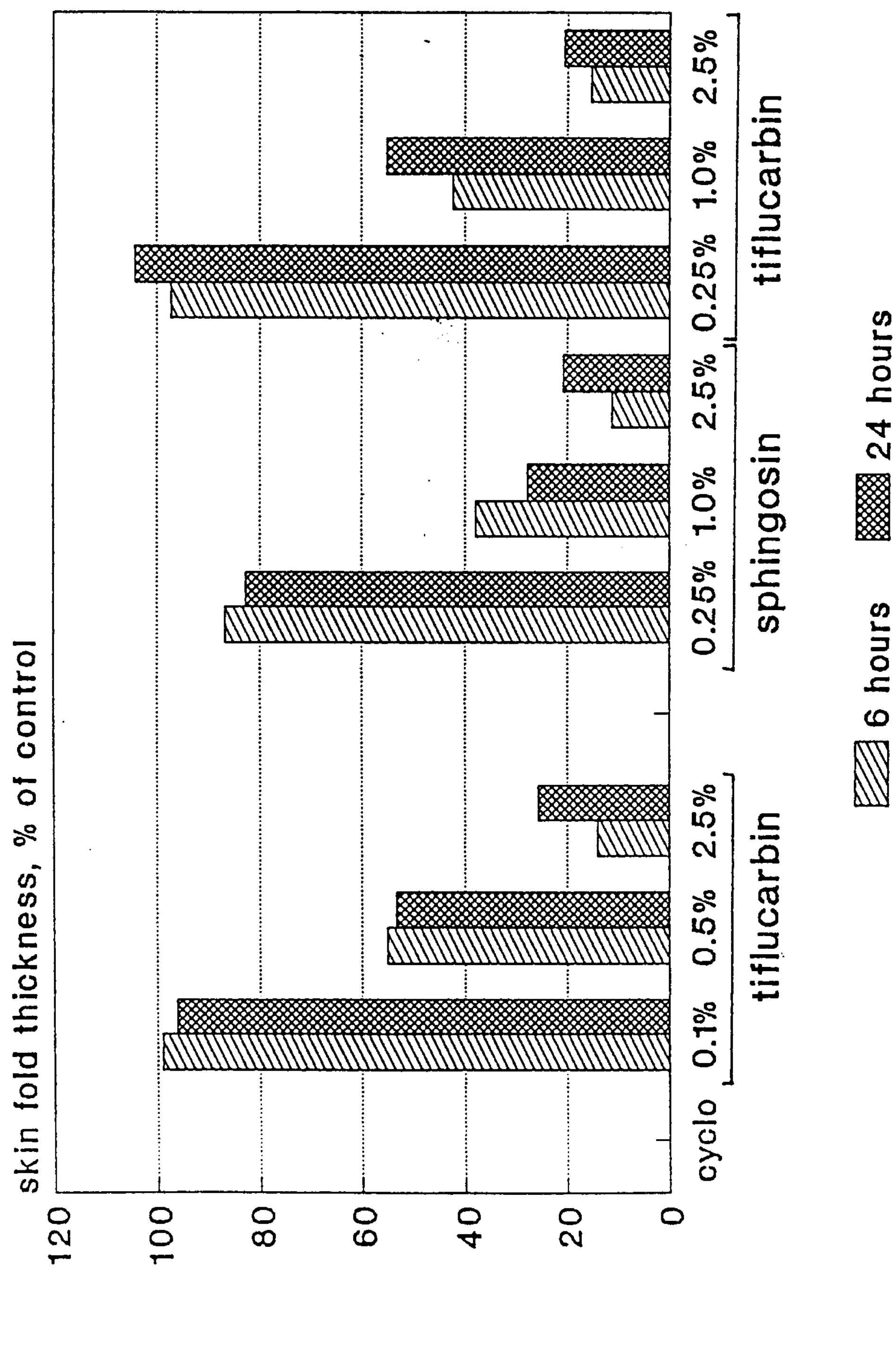

skin fold thickness, % of control
120
100
80
60
40
20
0
cyclo
0.1%
0.5%
2.5%
tiflucarbin
0.25%
1.0%
2.5%
sphingosin
0.25%
1.0%
2.5%
tiflucarbin
6 hours
24 hours

FIG. 14/15

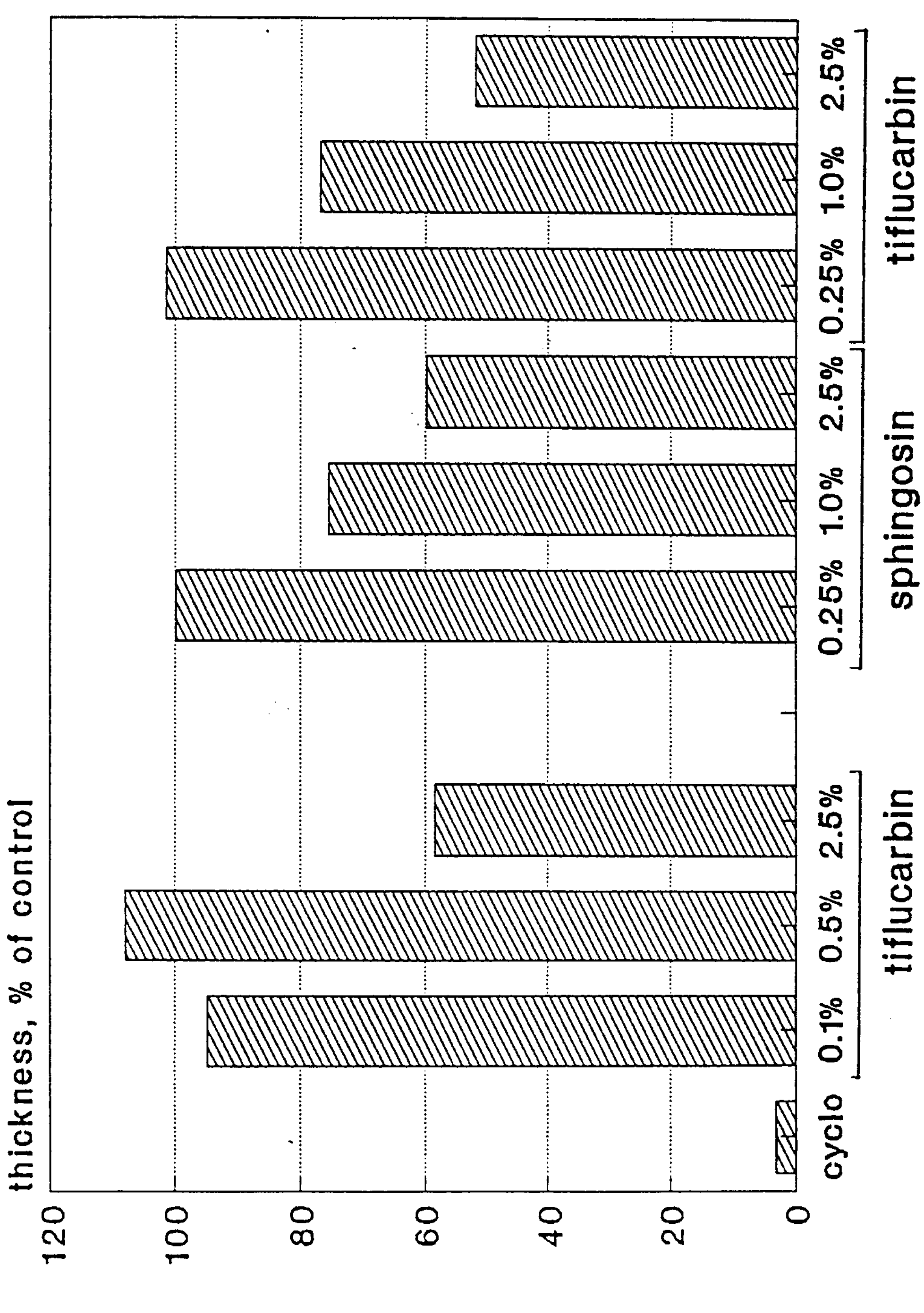
thickness, % of control
120
100
80
60
40
20
0
cyclo
0.1%
0.5%
2.5%
tiflucarbin
0.25%
1.0%
2.5%
sphingosin
0.25%
1.0%
2.5%
tiflucarbin

FIG. 15/15

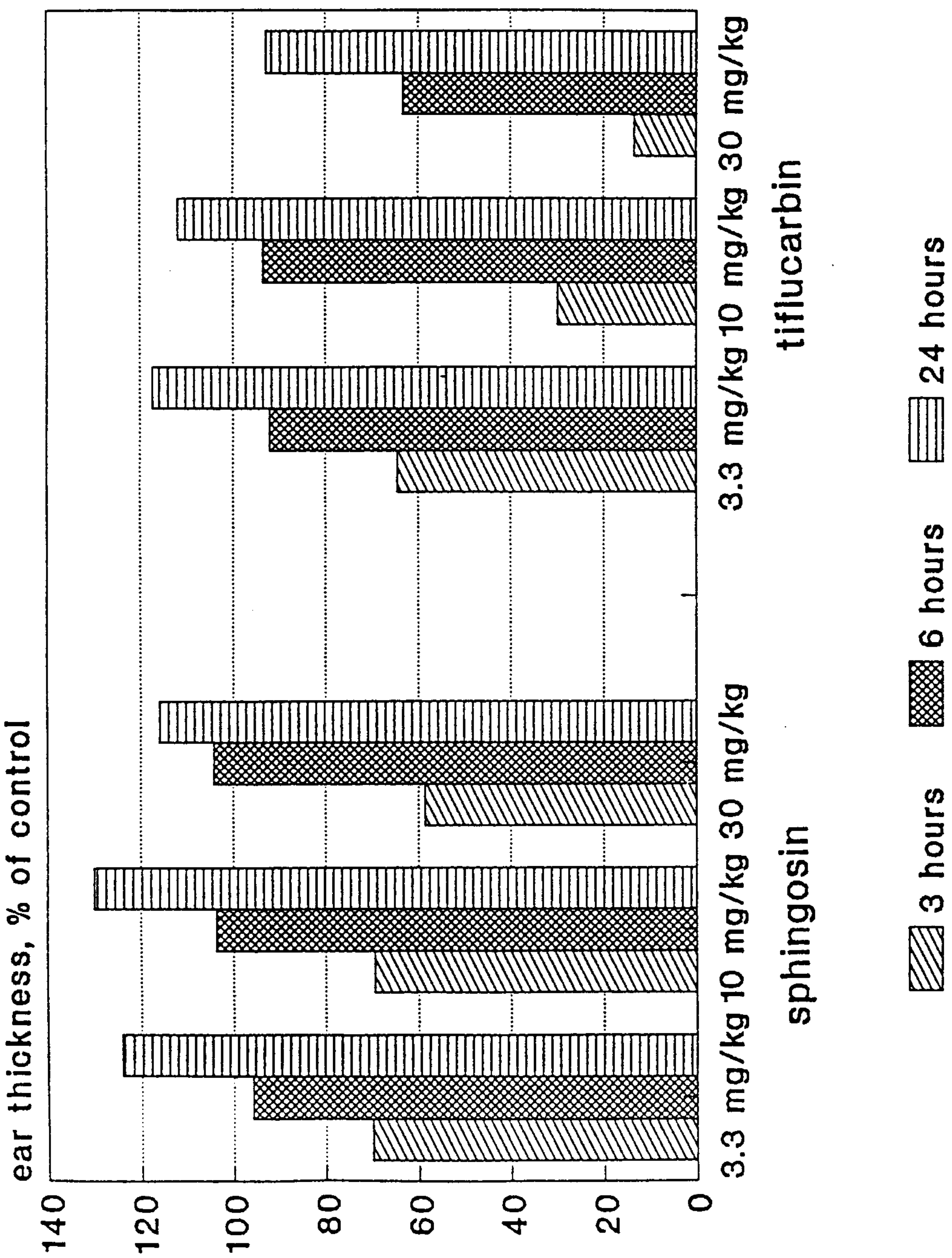
ear thickness, % of control
140
120
100
80
60
40
20
0
3.3 mg/kg 10 mg/kg 30 mg/kg
sphingosin
3.3 mg/kg 10 mg/kg 30 mg/kg
tiflucarbin
3 hours
6 hours
24 hours

European Patent Office

# EUROPEAN SEARCH REPORT

Application Number

EP 92 20 0429

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | ARCHIVES OF DERMATOLOGICAL RESEARCH, vol. 283, 1991, pages 456-460, Springer-Verlag; L. HEGEMANN et al.: "Effects of tiflucarbine as a dual protein kinase C/calmodulin antagonist on proliferation of human keratinocytes and release of reactive oxygen species from human leukocytes" * Whole document * | 1,2,4-8,10 | A 61 K 31/475 |
| X | ARZNEIMITTEL FORSCHUNG/DRUG RESEARCH, vol. 34, no. 10, 1984, pages 1254-1258; G. SCHÖLLNHAMMER et al.: "Biochemische Untersuchungen an Tienocarbin und verwandten Verbindungen | 5-8,10 | |
| X | DE-A-3 406 997 (TROPONWERKE GmbH) * Pages 21-26 * | 5-8,10 | |
| D,A | DE-A-2 854 014 (TROPONWERKE GmbH) * Claim 1; pages 27-31 * | 5-8,10 | TECHNICAL FIELDS SEARCHED (Int. Cl.5) |
| D,X | DE-A-3 311 342 (TROPONWERKE GmbH) * Pages 25-31 * | 5-8,10 | A 61 K |

The present search report has been drawn up for all claims

| Place of search | Date of completion of the search | Examiner |
|---|---|---|
| THE HAGUE | 11-05-1992 | KLAVER T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document